# EUROPÄISCHE PATENTSCHRIFT

(11) **EP 1 206 560 B1**
(45) Veröffentlichungstag und Bekanntmachung des Hinweises auf die Patenterteilung: **09.11.2005**
(21) Anmeldenummer: 00964009.5
(22) Anmeldetag: 10.08.2000
(51) Int. Cl.: C12N 15/82, C12N 15/55, A01H 5/00

(54) **PFLANZLICHE GENEXPRESSION UNTER DER KONTROLLE VON KONSTITUTIVEN PFLANZLICHEN V-ATPASE PROMOTOREN**
PLANT GENE EXPRESSION, CONTROLLED BY CONSTITUTIVE PLANT V-ATPASE PROMOTERS
EXPRESSION DE GENES VEGETAUX SOUS LE CONTROLE DE PROMOTEURS VEGETAUX CONSTITUTIFS DE V-ATPASE

(30) Priorität: 26.08.1999 DE 19940648; 26.08.1999 US 150887 P
(43) Veröffentlichungstag der Anmeldung: 22.05.2002
(73) Patentinhaber: BASF Plant Science GmbH, 67056 Ludwigshafen (DE)
(72) Erfinder: DUWENIG, Elke, 79102 Freiburg (DE); RAUSCH, Thomas, 69118 Heidelberg (DE)
(74) Vertreter: Isenbruck, Günter
(86) Internationale Anmeldenummer: PCT/EP2000/007774
(87) Internationale Veröffentlichungsnummer: WO 2001/014572

(56) Entgegenhaltungen:
- WO-A-97/30163
- WO-A-99/38977
- LEHR ANGELIKA ET AL: "cDNA and genomic cloning of sugar beet V-type H+-ATPase subunit A and c isoforms: Evidence for coordinate expression during plant development and coordinate induction in response to high salinity." PLANT MOLECULAR BIOLOGY, Bd. 39, Nr. 3, Februar 1999 (1999-02), Seiten 463-475, XP002166399 ISSN: 0167-4412 & DATABASE EMBL [Online] ACCESSION NO: Y11037, 4. April 1997 (1997-04-04) LEHR, A., ET AL.: "B.vulgaris BV-16/1 gene and promoter region" & DATABASE EMBL [Online] ACCESSION NO: Y11038, 4. April 1997 (1997-04-04) LEHR, A., ET AL.: "B.vulgaris BV-70/5 gene and promoter region"
- STRUVE I ET AL: "STRUCTURE AND FUNCTION OF THE PROMOTER OF THE CARROT V-TYPE PROTON ATPASE CATALYTIC SUBUNIT GENE" JOURNAL OF BIOLOGICAL CHEMISTRY, Bd. 265, Nr. 14, 1990, Seiten 7927-7932, XP002166400 ISSN: 0021-9258

## Beschreibung

Die Erfindung betrifft DNA-Konstrukte, die einen operativ mit einem heterologen Gen verbundenen pflanzlichen B. vulgaris V-ATPase Promotor Untereinheit c, Isoform 2 gemäß Seq. ID No. 1 umfassen. Die Erfindung betrifft weiterhin die Verwendung dieser Konstrukte in Form von Expressionskassetten, rekombinanten Vektoren sowie in transgenen Pflanzen, Pflanzenzellen oder Protoplasten.

Mittels gentechnischer Verfahren können Fremdgene gezielt in das Genom einer Pflanze übertragen werden. Dieser Prozeß wird als Transformation und die resultierenden Pflanzen werden als transgen bezeichnet. Bei der Expression von Fremdgenen in Pflanzen ist die Auswahl des Promotors oft ein kritischer Faktor. Während es wünschenswert sein kann, ein Gen nur als Antwort auf einen bestimmten abiotischen oder biotischen Stimulus hin zu exprimieren oder die Expression in einem bestimmten Gewebe zu lokalisieren, sollten andere Gene vorzugsweise konstitutiv exprimiert werden, d.h. in der gesamten Pflanze zu jedem Zeitpunkt und in allen Geweben.

Beispiele für eine durch einen bestimmten Stimulus induzierbare Expression sind die zum Beispiel in der EP 0 375 091 A1 bei der Kartoffel und in der WO 93/07279 beschriebene Wundinduktion, die in der WO 95/19443 beschriebene chemische Induktion (Ward et al. (1993) Plant Molecular Biology 22, 361-366), wie zum Beispiel die aus der EP 0 337 532 B1 bekannte Induktion mit Salicylat, die Lichtinduktion (Fluhr et al. (1986) Science 232, 1106-1112 und WO 91/05054 sowie eine temperaturabhängige Induktion, ebenfalls in der EP 0 337 532 B1 und in der Tomate in der WO 96/12814 beschrieben.

Beispiele für zell- und gewebespezifische Expression sind samen-, knollen- und fruchtspezifische (zusammengefaßt in Edwards und Coruzzi (1990) Annu. Rev. Genet. 24, 275-303 und US 5,753,475).

Des weiteren sind die phloemspezifische (Schmülling et al. (1989) Plant Cell 1, 665-670), die wurzelknöllchenspezifische (DE 3702497) sowie die meristemspezifische (Ito et al. (1994) Plant Molecular Biology 24, 863-878) Expression bekannt.

Promotoren, die eine konstitutive Expression der von ihnen kontrollierten Gene bewirken, können beispielsweise für die Selektion transformierter Pflanzenzellen (Expression eines selektierbaren Markergens in transgenen Pflanzen, Produktion von antibiotikaresistenten Pflanzenzellen) oder zur Erzeugung herbizidinsektizidtoleranter und pathogen-stressresistenter Pflanzen eingesetzt werden, da die Produkte der von ihnen kontrollierten Gene in allen Teilen der Pflanze vorliegen.

Fremdgene von weiterer agronomischer, medizinischer oder anderweitiger Bedeutung können in verschiedenen Pflanzen exprimiert werden, z.B. zur Produktion von heterologen rekombinanten Proteinen und zur Erzeugung von Pflanzen, die Säugerpolypeptide enthalten. Auch die Menge des räumlichen und zeitlichen Expressionsmusters endogener Pflanzengene kann mit Hilfe von konstitutiv aktiven Promotoren günstig verändert werden.

Der am häufigsten in der Pflanzengenetik verwendete konstitutive Promotor ist der virale 35S CaMV-Promotor (Franck et al. (1980) Cell 21, 285-294). Dieser Promotor enthält unterschiedliche Erkennungssequenzen für transkriptionale Effektoren, die in ihrer Gesamtheit zu einer konstitutiven Expression des eingeführten Gens führen (Benfey et al. (1989) EMBO J. 8,2195-2202).

Weitere konstitutive Promotoren viralen Ursprungs sind beispielsweise der in der EP 0 426 641 beschriebene Fig-wort Mosaic Virus Promotor, der Australian Banana-infecting Badnavirus (WO 99/00492) und der in der WO 99/09190 beschriebene Zuckerrohr Bacilliform Virus Promotor.

Pflanzeneigene konstitutive Promotoren sind beispielsweise der ALS Promotor aus Mais (WO 96/07746), der Aktin1-Promotor aus Reis (McElroy et al., Plant Cell 2, 163-171, 1990, US 5,641,876), der in der WO 91/13991 beschriebene Proline-Rich-Protein-Promotor aus Weizen, der DRU-Promotor aus Himbeere (WO 97/27307) und der H3 Histon-Promotor aus *Medicago sativa* (WO 97/20058).

Für eine Expression von Selektionsgenen und Resistenzgenen wäre es wünschenswert Promotoren zu haben, die eine starke, gleichmäßig konstitutive Aktivität in möglichst allen Pflanzengeweben bzw. Zelltypen aufweisen und außerdem unter Streßbedingungen noch stärker aktiv sind bzw. nicht reprimiert werden. Diese Promotoren sollten vorteilhafterweise nicht aus Pflanzenpathogenen (wie der 35S-CaMV-Promotor) stammen, dessen Expression auch in verschiedenen Pflanzengeweben unterschiedlich sein könnte.

Eine Regulierung des konstitutiven 35S-Promotors durch Streßfaktoren ist nicht beschrieben (häufig werden Elemente anderer streßinduzierter Promotoren mit dem CaMV 35S-Promotor gekoppelt); die meisten stressinduzierten Promotoren weisen unter Normalbedingungen nachteilhafterweise eine kaum nachweisbare Expression auf und werden nur unter den jeweiligen induzierenden Streßbedingungen stark induziert. Sie sind somit für viele Verwendungen ungeeignet.

Die WO 94/21793 beschreibt einen konstitutiven, durch Umweltbedigungen modulierten Promotor. Es handelt sich um einen konstitutiven Promotor aus Tabak, der durch Hitze- und Hormonschock, durch Verwundung, durch biotische Induktion und Infektion induziert wird. In der WO 94/21793 wird vorgeschlagen, diesen Promotor zur Selektion von Pflanzenschutzmitteln oder zum Schutz der Pflanze vor Streßsituationen zu verwenden.

Die EP 0 559 603 beschreibt als ein weiteres Beispiel den konstitutiven Promotor des Hitzeschockproteins hsp80 aus Blumenkohl. Teile dieses Promotors können auch bei heterologen, nicht-konstitutiven Promotoren, z.B. bei induzierbaren, anders regulierbaren oder inaktivierten Promotoren zu einer konstitutiven Expression führen. Die EP 0 559 603 beschreibt weiter, daß Insektenresistenz vermittelnde Gene, Herbizidresistenzgene, antimikrobielle Gene, antifungale Gene, antivirale Gene und anti-*feedant* Gene unter Kontrolle dieses Promotors stehen können. Der hsp80-Promotor besitzt eine sehr hohe konstitutive Aktivität, die unter Streßbedingungen jedoch nachteilhafterweise nur geringfügig erhöht wird.

In Zellen höherer Pflanzen spielt die V-type H⁺-ATPase (V-ATPase) eine zentrale Rolle, da sie maßgeblich den elektrochemischen H⁺-Gradienten am Tonoplasten bestimmt. Sie macht einen großen Anteil (7-35%) am Gesamttonoplastenprotein aus (Klink et al (1990) Bot Acta 103, 24-31; Fischer-Schliebs et al. (1997) Biol Chem 378, 1131-1139). Des weiteren findet sich die V-ATPase auch in den Membranen der Golgivesikel. Sie ist also auch in Zellen, die keine große Zentralvakuole enthalten, relativ stark exprimiert. Pflanzliche V-ATPasen sind aus mindestens 10 verschiedenen Untereinheiten zusammengesetzt, die in definierter Stöchiometrie ein Holoenzym von ca. 500.000 kDa bilden. Neben der oft an den gleichen Endomembranen exprimierten vacuoläre Pyrophosphatese (V-PPᵢase) spielt die V-ATPase eine zentrale Rolle für Prozesse wie Zellteilung, Zellstreckung und vakuoläre Metabolit- bzw. Salz-Akkumulation.

Untersuchungen zur Expressionskontrolle mehrerer V-ATPase-Gene in verschiedenen Pflanzen (Zuckerrübe, Tabak, Karotte, Mais, *Mesembryanthemum crystallinum*) haben gezeigt, daß zumindest einige V-ATPase-Gene hinsichtlich der Transkriptmengen eine koordinierte Expression zeigen (Rausch et al (1996) J Plant Physiol 148: 425-433; Löw et al (1996) Plant Physiol 110: 259-265; Löw und Rausch (1996) J Exp Bot 47: 1725-1732; Kirsch et al (1996) Plant Mol Biol 32: 543-547; Lehr et al., (1999) Plant Mol Biol 39: 463-475).

Der Erfindung liegt die Aufgabe zugrunde, neue DNA-Konstrukte umfassend pflanzliche konstitutive Promotoren zur Verfügung zu stellen, die verbesserte Eigenschaften gegenüber den bisher verwendeten pflanzlichen oder viralen Promotoren aufweisen und eine starke, konstitutive, durch Salzstreß und andere biotische oder abiotische Faktoren modulierte Genexpression in allen Pflanzenteilen ermöglichen. Insbesondere sollen die DNA-Konstrukte zur Expression von Selektionsmarkern und Resistenzgenen geeignet sein.

Diese Aufgabe wird durch ein DNA-Konstrukt gelöst, bei dem ein pflanzlicher B. vulgaris V-ATPase Promotor Untereinheit c, Isoform 2 gemäß Seq. ID No. 1 mit einem heterologen Gen operativ verbunden vorliegt. Erfindungsgemäß kann dieser pflanzliche V-ATPase Promotor auch ein deletierter oder Hybrid-V-ATPase Promotor der Untereinheit c, Isoform 2 sein, der weiterhin funktionell als Promotor aktiv ist.

Eine weitere Aufgabe der Erfindung ist es, einen neuen pflanzlichen konstitutiven Promotor der oben genannten Art zur Verfügung zu stellen.

Erfindungsgemäß wird diese Aufgabe durch ein die Sequenz des Promotors der *B*. *vulgaris* V-ATPase Untereinheit c Isoform 2 [SEQ ID Nr. 1] umfassendes Polynucleotid gelöst.

Zweckmäßige Weiterbildungen der Erfindung sind in den Unteransprüchen gekennzeichnet.

So kann das erfindungsgemäße DNA-Konstrukt einen auf andere Weise als den ersten Promotor regulierbaren zweiten Promotor umfassen, wodurch eine flexiblere Steuerung der Expression ermöglicht wird. Weiterhin kann mindestens ein weiterer Pyrimidinstretch in den Promotor eingefügt sein, der einen Einfluß auf die Promotoraktivität verursacht.

Bevorzugterweise ist das erfindungsgemäße DNA-Konstrukt eine Expressionskassette. Weiterhin ist bevorzugt, daß das heterolog exprimierte Gen ein Selektionsmarker oder ein Resistenz vermittelndes Gen ist. Beispiele für solche heterologen Gene sind insektizide Toxine (wie zum Beispiel von *Bacillius thuringiensis),* Herbizidresistenzgene, antimikrobielle Gene, antifungale Gene, antivirale Gene und anti-*feedant* Gene. Weitere geeignete Gene sind zum Beispiele selektierbare Gene, Reportergene oder Killergene. Selektierbare Gene sind zum Beispiel Antibiotikaresistenzgene wie Neomycin-Transferasegene, Hygromycin- Phosphotransferasegene oder Phosphoinothricin Acetyltransferasegene oder alternativ Herbizidresistenzgene wie Glufosinat-, Bromoxynil-, Sulfonamid- oder Glyphosatresistenzgene (weiteres in Glick und Thompson, Methods in Plant Molecular Biology and Biotechnology, CRC Press London, Kapitel 6.7, Seiten 71-119). Reportergene sind zum Beispiel Gene, die für Chloramphenicol Acetyltransferase (CAT), β-Glucoronidase (GUS), Luciferase (LUC), Green fluorescent protein (GFP), oder Thaumatin kodieren. Killergene sind zum Beispiel das Barnasegen, das TA29 Gen oder das Diphtheria ToxinGen (weitere Informationen dazu in "Transgenic Plants", E.Gaulun und A. Breiman, Imperial College Press, London, 1997).

Die Erfindung stellt weiterhin einen rekombinanten Vektor zur Verfügung, der ein erfindungsgemäßes DNA-Konstrukt umfaßt. Dieser Vektor kann auch ein Shuttle Vektor sein, wodurch seine Handhabung erleichtert wird.

In einer weiteren Ausführungsform der Erfindung ist der rekombinante Vektor dadurch gekennzeichnet, daß er ein Expressionsvektor ist. Weiterhin werden mit dem rekombinanten Vektor transformierte Mikroorganismen zur Verfügung gestellt, zum Beispiel ein transformiertes Agrobacterium tumefaciens.

Eine bevorzugte Ausführungsform der Erfindung betrifft eine transgene pflanzliche Zelle oder einen Protoplasten, dessen Genom ein erfindungsgemäßes DNA-Konstrukt umfaßt. Diese transgene pflanzlichen Zelle oder Protoplast können sowohl aus einer monokotylen als auch einer dikotylen Pflanze stammen. Bevorzugt sind dabei Zellen oder Protoplasten aus Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Besonders bevorzugt ist eine erfindungsgemäße transgene Pflanze, die dadurch gekennzeichnet ist, daß ihr Genom ein erfindungsgemäßes DNA-Konstrukt umfaßt. Die transgene Pflanze kann sowohl eine monokotyle wie auch eine dikotyle Pflanze sein. Besonders bevorzugt sind Pflanzen wie Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Die Erfindung stellt weiterhin ein Verfahren zur gesteuerten Expression eines heterologen Gens in einer Pflanzenzelle oder einem Protoplasten zur Verfügung, das eine starke, konstitutive, durch Salzstreß und andere abiotische oder biotische Faktoren modulierte Genexpression in allen Pflanzenteilen ermöglicht. Das erfindungsgemäße Verfahren ist dadurch gekennzeichnet, daß die Zelle oder der Protoplast zunächst mit einem erfindungsgemäßen DNA-Konstrukt, z.B. einer Expressionskassette transformiert wird und die so transformierte Zelle oder der Protoplast anschließend einem solchen biotischen oder abiotischen Streß ausgesetzt wird, daß die Expression des mittels des DNA-Konstruktes transformierten heterologen Gens effektiv gesteuert wird. Dieser Streß kann in Form von Salzstreß, z.B. durch NaCl oder KCl, Phosphatmangel, Stickstoffmangel, Saccharosemangel, Verwundung, Infektion, Temperatur, Trockenheit, Herbiziden oder mechanischer Belastung auftreten. Erfindungsgemäß können die für das Verfahren verwendeten Pflanzenzellen oder Protoplasten aus einer monokotylen oder dikotylen Pflanze stammen, bevorzugterweise aus Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Das erfindungsgemäße Verfahren kann auch zur gesteuerten Expression eines heterologen Gens in einer Pflanze verwendet werden. Dieses Verfahren ist dadurch gekennzeichnet, daß mit einem erfindungsgemäßen DNA-Konstrukt transformierte Zellen oder Protoplasten zu einer transgenen Pflanze regeneriert werden und die so erzeugte transgene Pflanze anschließend einem solchen biotischen oder abiotischen Streß ausgesetzt wird, daß die Expression des mittels des DNA-Konstruktes transformierten heterologen Gens effektiv gesteuert wird. Dieser Streß kann in Form von Salzstreß, z.B. durch NaCl oder KCl, Phosphatmangel, Stickstoffmangel, Saccharosemangel, Verwundung, Infektion, Temperatur, Trockenheit, Herbiziden oder mechanischer Belastung auftreten. Die für das Verfahren verwendete transgene Pflanze kann eine monokotyle oder dikotyle Pflanze sein, bevorzugt sind Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Die Erfindung stellt weiterhin ein Verfahren zur Verfügung, das die effektive Herstellung eines rekombinanten Proteins in einer Pflanzenzelle oder einem Protoplasten ermöglicht. Dabei wird die Zelle oder der Protoplast mit einem erfindungsgemäßen DNA-Konstrukt transformiert und die so transformierte Zelle oder der Protoplast anschließend einem solchen biotischen oder abiotischen Streß ausgesetzt wird, daß die Expression des mittels des DNA-Konstruktes transformierten rekombinanten Proteins erfolgt. Dieser Streß kann in Form von Salzstreß, z.B. durch NaCl oder KCl, Phosphatmangel, Stickstoffmangel, Saccharosemangel, Verwundung, Infektion, Temperatur, Trockenheit, Herbiziden oder mechanischer Belastung auftreten. Das so hergestellte Protein kann anschließend aus der Pflanzenzelle oder dem Protoplasten nach dem Fachmann bekannten Methoden isoliert werden. Die für das Verfahren verwendeten Pflanzenzellen oder Protoplasten können aus einer monokotylen oder dikotylen Pflanze stammen. Besonders bevorzugt sind Pflanzenzellen oder Protoplasten aus Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Die Erfindung stellt weiterhin ein Verfahren zur Verfügung, das die effektive Herstellung eines rekombinanten Proteins in einer Pflanze ermöglicht. Das Verfahren ist dadurch gekennzeichnet, daß mit einem erfindungsgemäßen DNA-Konstrukt transformierte Zellen oder Protoplasten zu einer transgenen Pflanze regeneriert werden und die so erhaltene transgene Pflanze anschließend einem solchen biotischen oder abiotischen Streß ausgesetzt wird, daß die Expression des mittels des DNA-Konstruktes transformierten rekombinanten Proteins erfolgt. Dieser Streß kann in Form von Salzstreß, z.B. durch NaCl oder KCl, Phosphatmangel, Stickstoffmangel, Saccharosemangel, Verwundung, Infektion, Temperatur, Trockenheit, Herbiziden oder mechanischer Belastung auftreten. Das so hergestellte Protein kann anschließend aus der Pflanze isoliert werden. Die für das Verfahren verwendeten Pflanzen können monokotyle oder dikotyle Pflanzen sein. Besonders bevorzugt sind Pflanzen wie Zuckerrübe, Tabak, Gerste, Reis, Kartoffel, Sonnenblume, Soja, Tomate, *Canola,* Weizen, Raps, Sorghum, Karotte, Mais, *Mesembranthemum crystallinum* oder *Arabidopsis thaliana*.

Die vorliegende Erfindung umfaßt weiterhin die Verwendung eines erfindungsgemäßen DNA-Konstrukts zur Herstellung eines rekombinanten Proteins in einer Pflanze, Pflanzenzelle oder einem Protoplasten. Die Erfindung umfaßt außerdem nach einem der erfindungsgemäßen Verfahren hergestellte rekombinante Proteine.

Das erfindungsgemäße DNA-Konstrukt kann auch zur Expression eines Gens in einer Pflanze unter biotischem oder abiotischem Streß verwendet werden. Weiterhin kann der erfindungsgemäße pflanzliche B. vulgaris V-ATPase Promotor Untereinheit c, Isoform 2 [Seq. ID No. 1] zur Expression eines Gens in einer Pflanze unter biotischem oder abiotischem Streß verwendet werden. Dieser Promotor kann auch in Form eines deletierten oder Hybrid-V-ATPase Promotors verwendet werden. In dem verwendeten Promotor kann weiterhin mindestens ein weiterer Pyrimidinstretch eingefügt sein.

Ein weiterer Gegenstand der Erfindung ist eine mit einem erfindungsgemäßen DNA-Konstrukt transformierte und gegen biotischen oder abiotischen Streß resistente Pflanzenzelle oder Protoplast. Dieser Streß kann in Form von Salzstreß, z.B. durch NaCl oder KCl, Phosphatmangel, Stickstoffmangel, Saccharosemangel, Verwundung, Infektion, Temperatur, Trockenheit, Herbiziden oder mechanischer Belastung auftreten. Bevorzugt ist eine transformierte salzstreßresistente Pflanzenzelle oder Protoplast.

Die Erfindung umfaßt weiterhin eine mit einem erfindungsgemäßen DNA-Konstrukt transformierte und gegen biotischen oder abiotischen Streß resistente Pflanze, die bevorzugt eine transformierte salzstreßresistente Pflanze ist.

Aus einer genomischen Bank für *Beta vulgaris* wurden insgesamt drei verschiedene V-ATPase-Gene isoliert. Die cDNA-Klone und genomische Klone für die Untereinheiten A und c1, welche dem peripheren V1-Komplex und dem membran-integriertem V0-Komplex entsprechen, sind bereits beschrieben worden (Lehr et al, Plant Molecular Biology 39, 463-475, 1999, GenBank/EMBL-Datenbank: X98767, X98851, Y11038, Y11037).

RNA Blot Analysen mit genspezifischen cDNA-Sonden zeigen eine Expression der klonierten Untereinheit A und c1, sowie der im Rahmen der vorliegenden Erfindung klonierten Isoformen c2 in Wurzeln, Blättern und Zuckerrübensuspensionskultur.

Es stellte sich dabei überraschenderweise heraus, daß alle drei Promotoren eine sehr hohe Aktivität zeigen, in der Regel höher als die des 35S CaMV-Promotor.

Dafür scheinen in den Promotoren vorhandene Polypyrimidinsequenzen wichtig zu sein. Die Isoformen A und c1 weisen je zwei, die Isoform c2 nur einen solchen Sequenzbereich auf.

Im Rahmen der vorliegenden Erfindung konnte nun überraschenderweise gefunden werden, daß sich der pflanzliche B. vulgaris V-ATPase-Promotor Untereinheit c, Isoform 2 [Seq. ID No. 1] hervorragenderweise zur Konstruktion von DNA-Konstrukten eignet, die bevorzugt als pflanzliche Expressionskassetten verwendet werden können.

Diese Expressionskassetten können dazu verwendet werden, eine Expression von heterologen Proteinen in der Pflanze zu bewirken, die durch Umwelteinflüsse gesteuert ist. Die Erfindung ermöglicht es somit, die Pflanze oder Pflanzenzelle mit einem oder sogar mehreren heterologen Genen auszustatten, die bei "Bedarf" (bei Änderung der Umwelt) für die Pflanze in der Pflanze zusätzlich zu ihrer konstitutiven Expression induziert werden.

Luciferase-Reportergenstudien mit den Promotoren V-ATPase A und c1 aus Zuckerrübe und dem 35S-Promotor an Zuckerrübenzellkulturen sind unter Salzstreß durchgeführt worden (Lehr et al, Plant Mol Biol 39, 463-475, 1999). Dabei konnte eine Induktion der Promotoraktivität für die Isoformen A und c1 nachgewiesen werden. Die zum Vergleich untersuchte 35S CaMV-Promotoraktivität wird durch Salz nicht induziert und sogar reprimiert. Im Rahmen der Erfindung konnte eine überraschende Induktion durch 100 mM NaCl oder 100 mM KCl für alle drei Promotoren nachgewiesen werden, obwohl sich alle drei Promotoren in der DNA-Sequenz unterscheiden.

Überraschenderweise konnte weiter gefunden werden, daß bei den erfindungsgemäßen Promotoren keine Verringerung der Promotoraktivität bei Phosphatmangel/Nährstoffmangel auftritt, im Gegensatz zum 35S-CaMV-Promotor. Die konstitutive Expression bleibt somit idealerweise stabil.

Hingegen tritt eine Induktion der Promotoraktivität durch Verwundung auf, der 35S-CaMV-Promotor wird unter vergleichbaren Bedingungen nicht induziert. Eine Beeinflussung der Promotoraktivität durch Saccharosemangel konnte ebenfalls festgestellt werden, er reduziert die Aktivität von c1, nicht jedoch die des 35S-CaMV-Promotors.

Weiterhin konnte eine Beeinflussung der Promotoraktivitäten durch abiotische Faktoren, wie hohe (30-35°C) oder niedrige (2-5°C) Temperatur nachgewiesen werden.

Es konnte zudem gefunden werden, daß verschiedene Deletionen in diesen Promotor eingeführt werden können und das dieser Promotor dadurch entweder a) gesteigerte Aktivität, b) im wesentlichen identische Aktivität wie der native Promotor, c) verringerte Aktivität, d) höhere Induzierbarkeit unter Streßbedingungen als der native Promotor oder e) geringere Induzierbarkeit unter Streßbedingungen als der native Promotor aufwies.

"Operativ verbunden" bedeutet Anordnung auf solche Weise, daß die normale Funktion der Komponenten durchgeführt werden kann. Deshalb bedeutet eine "operativ" mit einer Kontrollsequenz "verbundene" kodierende Sequenz eine Konfiguration, in der die kodierende Sequenz unter der Kontrolle der Sequenzen exprimiert werden kann.

Der Begriff "Kontrollsequenzen" bezeichnet DNA-Sequenzen, die zur Expression einer operativ verbundenen kodierenden Sequenz in einem *host*-Organismus erforderlich sind. Die zum Beispiel für Prokaryonten geeigneten Kontrollsequenzen umfassen einen Promotor, gegebenenfalls eine Operatorsequenz, eine Ribosemenbindungsstelle und möglicherweise weitere bis jetzt wenig verstandene andere Sequenzen. Eukaryontische Zellen sind dafür bekannt, Promotoren, Polyadenylierungssignale und *Enhancer* zu enthalten.

Der Begriff "Gen" bezieht sich auf eine DNA-Sequenz, die für ein isolierbares bioaktives Polypeptid oder einen Precursor kodiert. Das Polypeptid kann durch eine Sequenz in voller Länge oder jeden Teil der kodierenden Sequenz kodiert werden, solange die enzymatische Aktivität erhalten bleibt.

Ein Aspekt der Erfindung ist somit ein DNA-Konstrukt, umfassend einen pflanzlichen B. vulgaris V-ATPase Promotor Untereinheit c, Isoform 2 [Seq. ID No. 1], operativ verbunden mit einem heterologen Gen. Es ist bekannt, daß kleine Veränderungen in der Sequenz des Promotors vorhanden sein können, zum Beispiel durch die Degenerierung des genetischen Codes, ohne daß dessen Aktivität wesentlich verändert wird. Solche Promoter-Sequenzen werden als "funktionale Äquivalente" des pflanzlichen B. vulgaris V-ATPase Promotors, Untereinheit c, Isoform 2 [Seq. ID No. 1] bezeichnet.

Mit dem Begriff "funktionale Äquivalente" sind alle DNA-Sequenzen bezeichnet, die komplementär zu einer DNA-Sequenz sind, die unter stringenten Bedingungen mit der Referenzsequenz hybridisieren und eine zu einem pflanzlichen V-ATPase Promotor ähnliche Aktivität aufweisen.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

"Heterologe Gene" sind DNA-Sequenzen, die für Peptide oder Proteine kodieren, die keine pflanzlichen V-ATPase-Untereinheiten A, c1 oder c2 sind.

Ein "deletierter oder Hybrid-V-ATPase Promotor Untereinheit c, Isoform 2 [Seq. ID No. 1]" ist jeder V-ATPase Promotor Untereinheit c, Isoform 2 [Seq. ID No. 1], der eine Deletion aufweist oder in seinem Aufbau verändert ist und immer noch Promotoraktivität aufweist.

Die Erfindung soll im folgenden in Zusmmenhang mit den Zeichnungen weiter beschrieben werden.

### I. Methode zur Gewinnung der genomischen Bank für Beta vulgaris L.

### Herstellung einer genomischen Bank

Es wurde eine genomische Bank aus Blattmaterial von *Beta vulgaris* L. Typ 3A39111 im "Lambda Fix II/Xho I partial Fill-in" Vektor der Firma Stratagene angelegt.

Die Verwendung des "Lambda Fix II/Xho I partial Fill-in Kits" ermöglicht eine effiziente Klonierung genomischer DNA Fragmenten ohne die zeitaufwendige Größenfraktionierung über ein Agarosegel. Die genomische DNA wird hierfür mit der Restriktionsendonuklease Sau3 AI partiell verdaut und die freien Enden mit Klenow Polymerase, unter Einbau von dGTP sowie dATP aufgefüllt. Die hieraus resultierenden 3'-AG-5' Überhänge verhindern eine Selbstligierung der genomischen DNA Fragmente. Ebenso wird auf Grund der 3'-CT-5' Überhänge des Vektors (Xho I verdauter Vektor, partiell aufgefüllte Enden mit dCTP, dTTP) eine Religierung des zentralen Vektorfragmentes ("stuffer element") unterbunden. Eine Amplifizierung von Wildtyp Lambda Fix II Phagen (enthaltend das "stuffer element") wird zudem durch Verwendung des P2 lysogenen E. coli Stammes XLI-Blue MRA(P2) verhindert.
Das verwendete Verfahren ermöglicht somit eine hohen Ausbeute an rekombinanten genomischen Klonen im gewünschten Größenbereich.

### Partieller Verdau genomischer DNA

Durch einen Testverdau wird die optimale Enzymkonzentration zur Gewinnung von Fragmenten in dem erforderlichen Größenbereich von 15-23 kb ermittelt. Einzelne Reaktionen werden hierfür gemeinsam mit einem DNA-Marker im niederprozentigen Agarosegel aufgetrennt und die mittleren Fragmentgrößen bestimmt. Da es bei der elektrophoretischen Auftrennung insbesondere im Bereich hochmolekularer DNA zur Verzögerung im Laufverhalten kommt, kann über die maximale Fluoreszenzintensität keine unmittelbare Bestimmung der Fragmentgröße erfolgen. Vielmehr wird nach Seed et al. (1982) die Größe der Fragmente um den Faktor 2 überschätzt. Zur Gewinnung von Fragmenten im gewünschten Größenbereich wird daher im präparativen Verdau lediglich die Hälfte der über die Gelelektrophorese bestimmten optimalen Enzymkonzentration eingesetzt.

### Testverdau

Für den Testverdau wird das Restriktionsenzym Sau3A I (10 U/µl, Promega) im Restriktionspuffer [1x Puffer B (Promega) supplementiert mit 0.1 mg/ml acetyliertes BSA (Sigma)] auf die Konzentrationen 0,1 U/µl, 0,05 U/µl, 0,025 U/µl, 0,01 U/µl] verdünnt und Enzymlösungen auf Eis gelagert. 25 µg CsCl₂-gereinigte, in TE-Puffer gelöste, genomische DNA werden in einem Gesamtvolumen von 1,125 ml Restriktionspuffer (s.o.) resuspendiert und als Aliquots ä 225 µl in Reaktionsgefäßen auf Eis aufbewahrt. Die DNA-Lösungen (je 5 pg DNA) werden für ca. 20 Minuten im Wasserbad bei 37°C vorgewärmt, und die einzelnen Reaktionen werden durch das Hinzufügen von 25 µl der Restriktionsenzymlösung gestartet. Einer Kontrollprobe werden hierbei 25 µl reiner Pufferlösung hinzupipettiert. Der Verdau erfolgt für 2 Minuten bei 37°C und wird durch hinzufügen von je 10 µl 0,5 M Na₂-EDTA gestoppt. Die DNA wird hierauf unter Zusatz von 1 Volumen Isopropanol und 0,1 Volumen 3 M Na-Acetat (pH 7) gefällt, mit 70 %igem Ethanol gewaschen und im Anschluß an das Trocknen in je 10 µl TE-Puffer resuspendiert.
Die elektrophoretische Auftrennung der Ansitze erfolgt über Nacht im Ethidiumbromid-gefärbten 0,5%igen TAE-Gel (19 cm Lauflänge), über Nacht, bei 2 Volt/cm und RT. Als Größenmarker wurde in der Untersuchung Hind III verdaute Lambda DNA sowie die 1 Kb DNA *ladder* (GIBCO, BRL) verwendet.

### Präparativer Verdau

Die Bedingungen für den präparativen Verdau entsprechen im wesentlichen denen des Testansatzes. Zur Standardisierung werden DNA-Konzentration, Gesamtvolumen der Reaktion, sowie Zeit beibehalten. Die Enzymkonzentration ergibt sich aus dem Testverdau.
100 µg genomische DNA wird in Aliquots verdaut (s.o.) und nach Zugabe von 0,11 Volumen 10x STE sowie 0,3 Volumen 1x STE zweimalig mit 1 Volumen Phenol/Chloroform/Isoamylalkohol (25:24:11, v/v/v) extrahiert. Die Präzipitation erfolgt nach Zugabe von 0,5 Volumen 7,5 M Na-Acetat, 2 Volumen absolutem Ethanol für 30 Minuten bei -20 °C. Die DNA wird mit 70 %igem Ethanol gewaschen, bei Raumtemperatur getrocknet und unter Vereinigen der Pellets in 200 µl TE-Puffer resuspendiert. Alle Zentrifugationsschritte erfolgen für 10 Minuten, bei 12.000 g und 4 °C. Der Verdau wird bei -20 °C gelagert.

### 10x STE: 0,1 M NaCl, 10 mM Tris-HCl, pH 8, 1 mM Na₂-EDTA

### Klenow Reaktion

50 µg der partiell verdauten genomischen DNA werden in einem Gesamtvolumen von 350 µl 1x Fill-In Puffer (1x Klenow-Puffer: 50 mM Tris-HCl, pH 7,2, 10 mM MgSO₄, 0,1 mM DTT, Promega; 0,166 mM dGTP, 0,166 mM dATP) mit 15 U Klenow Polymerase (5 U/µl, Stratagene) behandelt. Die Reaktion wird durch Zufügen von 0,5 Volumen STE-Puffer (0,1 M NaCl, 10 mM Tris-HCl, pH 8, 1 mM Na₂-EDTA, pH 8) gestoppt, und die DNA hierauf phenolisiert und chloroformiert. Nach Zusatz von 0,5 Volumen Na-Acetat, 2 Volumen absolutem Ethanol wird die DNA für 30 Minuten bei -70 °C gefällt, für 15 Minuten bei 12.000 g und 4 °C pelletiert und in 70 %igem Ethanol gewaschen. Das getrocknete Pellet wird in TE-Puffer resuspendiert und bei -20°C gelagert.

### Ligation in den Lambda FIX II/Xho I partial Fill-in Vektor

In einem Reaktionsvolumen von 10 µl werden 1 µg Lambda DNA (1 µg/µl Lambda FIX II/Xho I partial Fill-in Vektor) mit 0,4 µg genomischer DNA Fragmente in Ligasepuffer (10 mM Tris-HCL, pH 7,8, 10 mM MgCl₂, 10 mM DTT, 1 mM rATP., Promega) auf Eis resuspendiert und unter Zusatz von 3,75 U T4 DNA Ligase (15 U/µl, Promega) über Nacht bei 6-8 °C inkubiert. Zur Untersuchung der Ligationseffizienz werden in einem Kontrollansatz 0,3 µg Testinsert (50 ng/µl, 12-kb pMF Testinsert; BamH I Fragment partiell aufgefüllt mit dATP, dGTP) mit 1 µg Lambda DNA unter den oben genannten Bedingungen ligiert. Die Verpackung der DNA erfolgt am darauffolgenden Tag.

### Verpackung der Lambda DNA

Zur Herstellung einer repräsentativen genomischen Bank wird die benötigte Gesamtanzahl unabhängiger Klone nach der von Clarke und Carbon (1976) beschriebenen Formel berechnet. Aliquots der Ligationsreaktion werden verpackt, und über die Titerbestimmug wird die Anzahl unabhängiger Klone pro Verpackungsreaktion ermittelt. Falls erforderlich, werden weitere Verpackungsreaktionen durchgeführt. Insgesamt sollte die berechnete Mindestanzahl an unabhängigen Klonen erreicht werden.
Für die Verpackung der Ligationsprodukte wurde der Gigapack III Gold packaging extract der Firma Stratagene verwendet. Ein Verpackungsextrakt (50 µl) wird in der Hand zum Antauen gebracht und nach Zugabe von 1 µl Ligationsansatz auf Eis gelagert. Zur Kontrolle der Verpackungseffizienz werden 0,2 µg Lambda Kontroll DNA (wild-type cl857 Sam7, 0,2 µg/µl) ebenso verpackt. Der Extrakt wird mit einer Pipette vorsichtig resuspendiert und hierauf für 2 Stunden bei Raumtemperatur inkubiert. Die Reaktion wird durch Zugabe von 500 µl SM Puffer (0,01 % Gelatine, 50 mM Tris-HCl, pH 7,5, 100 mM NaCl, 8 mM MgSO₄) sowie 20 µl Chloroform gestoppt und die Lösung durch vorsichtiges Schwenken gemischt. Im Anschluß erfolgt eine Zentrifugation für 1 Minute, bei 16.000 g und 4 °C. Der Überstand wird in ein Reaktionsgefäß (überführt und bis zur Verwendung bei 4 °C gelagert. Das Anlegen der Titerplatten erfolgt noch am gleichen Tag.
Es wurden folgende Verdünnungsstufen der einzelnen Verpackungsextrakte verwendet:

| Verpackungsextrakt | Verdünnungsstufe |
|---|---|
| Ligation der *B. vulgaris* genom. DNA | 1 X 10⁰ /1 X 10⁻¹ |
| Ligation des Kontroll-Inserts (pMF) | 1 X 10⁻¹ /1 X 10⁻² |
| Kontroll DNA (wild-type c1857 Sam7) | 1 X 10⁻³ /1 X 10⁻⁴ |

### Amplifizierung der genomischen Bank

Zur Gewinnung einer hoch konzentrierten Phagensuspension, welche als stabile Stammkultur bei -80 °C gelagert werden kann, wurde die genomische Bank von *B. vulgaris* amplifiziert. Die für die Amplifikation benötigte Mindestanzahl unabhängiger Klone sollte hierbei das gesamte Genom repräsentieren (Clarke und Carbon, 1976).

Die Amplifizierung erfolgt in Form von Plattenlysaten (150 mm ∅ LB-Agarplatte). Die benötigte Menge an Verpackungsextrakt richtet sich nach der Mindestanzahl unabhängiger Klone (s.o.). Pro Plattierungsansatz werden 600 µl kompetenter Wirtszellen mit je 50.000 pfu infiziert und für 15 Minuten bei 37 °C unter leichtem Schütteln inkubiert. Für das Ausplattieren wird 6 ml Top-Agar verwendet. Die Inkubationszeit beträgt bei 37 °C etwa 8 Stunden. Die Größe der Plaques sollte einen Durchmesser von 1-2 mm erreichen. Anschließend werden die Platten mit 10 ml SM-Puffer überschichtet und über Nacht auf einem Taumelschüttler bei niedrigster Umdrehung im Kühlraum inkubiert. Die Überstände werden in ein verschließbares Zentrifugengefäß überführt, die Platten mit 2 ml SM-Puffer abgespült und die Überstände vereinigt. Nach Zugabe von Chloroform (5% Endkonz.) wird die Phagensuspension für 15 Minuten bei Raumtemperatur inkubiert und durch Zentrifugation für 10 Minuten bei 2000 g von Bakterienrückständen getrennt. Der überstand wird in ein steriles Gefäß überführt, mit Chloroform (3 % Endkonz.) versetzt und kann so für mehrere Monate bei 4 °C gelagert werden. Im Anschluß an eine Titerbestimmung der amplifizierten genomischen Bank werden Aliqouts in 7 % DMSO (Dimethyisuifoxid) als Stammlösung bei -80 °C gelagert. Für die Titerbestimmung (90 mm ∅ LB-Platten) werden folgende Verdünnungsstufen eingesetzt: 1 x 10⁻⁶, 1 X 10⁻⁷, 1 X 10⁻⁸.

### II. Methoden zur Klonierung von Promotor-Reportergen-Konstrukten für die V-ATPase-Promotoren A, c1 und c2, mit den Reportergenen Luciferase (bzw. β-Glucuronidase)

### Subklonierung von genomischen Klonen mit Promotorregionen

Ein einzelner Typ von genomischen Klonen wurde für die Untereinheit A erhalten, während für Untereinheit c Klone zweier unterschiedlicher Isoformen isoliert wurden. Zur Sequenzanalyse des Klons der genomischen Untereinheit A wurde ein mit der Untereinheit A cDNA hybridisierendes 4,3 kb EcoRI Fragment (pBVA/70) in den pBluescript II SK+ Vektor subkloniert. Für Untereinheit c wurden ein 8 kb XbaI/HindIII Fragment (pBVA/16-1) und ein 5 kb EcoRV/HindIII Fragment in denselben Vektor subkloniert.

### Klonierung von Promotor-Reportergen Konstrukten

### BVA/70 Promotor

Ein 1,3 kb HpaII Fragment des pBVA/70 Klons wurde mit Klenow Polymerase mit *blunt*-Enden versehen und in die SmaI-Stelle des pBluescript Vektors ligiert, um so pBVA/70 zu erhalten. Restriktion mit HindIII und BglII ergab ein Promotorfragment von 1,3 kb mit seinem 3'-Ende bei Position -28 in bezug auf den Transskriptionsstart. Um die Promotor-GUS Fusion zu erhalten, wurde das HindIII/BglII an die BamHI/EcoRI GUS Kassette von pBI221 (Clontech) ligiert und danach in den pBluescript Vektor kloniert um so pBVA/70p-GUS zu erhalten. Um die Promotor-LUC Fusion zu erhalten, wurde ein PstI/BgIII Promotorfragment vor die BamHI/HindIII Kassette von pCaMVLN ligiert und in den pBluescript Vektor kloniert.

### BVA/16-1 Promotor

Die BamHI/EcoRI GUS Kassette von Plasmid pBI221 (Clontech) wurde in pBluescript kloniert (pBGUS), aus dem es mit BamHI und SalI herausgeschnitten wurde. Ein 1,3 kb PstI/BglII-Promotorfragment von pBVA/16-1 mit seinem 3'-Ende bei Position -40 in bezug auf den Transskriptionsstart wurde an die GUS-Kassette ligiert und in den PstI/SalI-verdauten pBluescript kloniert, um so pBVA/16-1p-GUS zu erhalten. Die Klonierung der Promotor-LUC Fusion folgte demselben Verfahren wie für BVA/70 beschrieben (siehe oben).
Plasmide pFF19G und pCaMVLN wurden jeweils als Standards für GUS und LUC verwendet.

### Charakterisierung der Promotor/Reportergen-Konstrukte

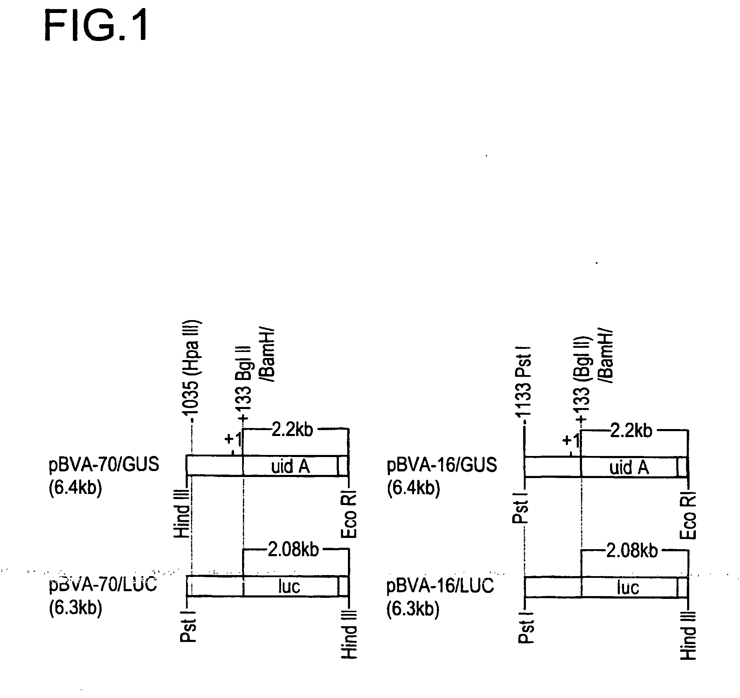

Für einen Vergleich der relativen Promotoraktivitäten der Untereinheiten A und c1 der V-Typ H⁺-ATPase aus *Beta vulgaris* wurden etwa 1,2 kb große Fragmente aus dem Bereich stromaufwärts der kodierenden Region der genomischen Klone pBVA-70 und pBVA-16/1 isoliert. Der Begriff Promotor bezeichnet in diesem Zusammenhang den 5'-regulativen Bereich und somit sowohl die Promotorregion als auch Teile des 5'-nichttranslatierten leaders. Diese Fragmente wurden im Anschluß vor die entsprechenden (s.u.) Reportergen-Kassetten in den Phagemidvektor pBlueskript II SK+ ligiert. Als Reportergen wurde neben dem β-Glucuronidase Strukturgen aus *Escherichia coli* (uid A) das aus *Photinus pyralis* stammende Luciferase Gen verwendet (Jefferson, 1987; de Wet et al., 1987). Die Promotor/Reportergen-Konstrukte stellen "Transkriptionsfusionen" dar, da sich die 3'-Enden der eingesetzten Promotorfragmente jeweils 30-40 bp oberhalb der Translationsstartstelle der genomischen Klone befinden. Die Figur 1. zeigt eine schematische Darstellung der einzelnen Konstrukte. Die Expressionsvektoren werden im folgenden als pBVA-70(bzw.16)/GUS sowie pBVA-70(bzw.16)/LUC bezeichnet.

### Charakterisierung des Promotorfragmentes aus pBVA-70

Die Gesamtgröße dieses Fragmentes beträgt 1,237 kb. Es umschließt den Bereich zwischen den Positionen -1035 bis +202, relativ zur Transkriptionsstartstelle (+1) des genomischen Klones pBVA-70, und enthält somit neben dem Promotor den Großteil der angrenzenden Leaderregion (Gesamtlänge des Leaders = 230 bp).

### Charakterisierung des Promotorfragmentes aus pBVA-16/1

Die Gesamtgröße dieses Fragmentes beträgt 1,265 kb. Es umfaßt den Bereich zwischen den Positionen -1130 bis +135, relativ zur Transkriptionsstartstelle (+1) des Klons pBVA-16/1, und beinhaltet somit neben der Promotorsequenz den Großteil der Leaderregion (Gesamtlänge des Leaders = 174 bp).
Figur 1 zeigt eine schematische Darstellung der Konstrukte pBVA-70(16)/GUS und pBVA-70(16)/LUC. Fragmente (ca. 1,25 kb) aus dem Bereich der 5'-nichttranslatierten Region der genomischen Klone pBVA-70 sowie pBVA-16/1 wurden in den Phagemidvektor pBlueskript II SK+ vor die jeweilige Reportergenkassette ligiert. Für die Herstellung der Promotor-GUS Konstrukte wurde die BamHI/EcoRI Kassette des Vektors pBI-221 der Firma Clontech verwendet. Sie trägt neben dem β-Glucoronidase Strukturgen aus *E. coli* die Polyadenylierungsregion des Nopalinsynthasegens (Ti-Plasmid, *Agrobacterium tumefaciens*). Die Gewinnung der Promotor-LUC Konstrukte erfolgte unter Verwendung der BamHI/HindIII Kassette des Vektors pCaMVLN. Sie enthält das Luciferase Strukturgen von *Photinus pyralis* sowie die Polyadenylierungsregion des Nopalinsynthasegens. Die Angaben in Zahlen bezeichnen die Position der Schnittstellen der verwendeten Promotorfragmente bezogen auf die Trannskriptionsstartstellen (+1) der genomischen Klone.

### Herstellung eines BVA/16-2 Promotor-LUC-Konstruktes

Als weiteres Reportergen zur Herstellung eines Konstruktes wurde das aus *Photinus pyralis* stammende Luciferase Gen eingesetzt (de Wet et al. 1987), EMBL Acc. no. M 15077. Zur Herstellung eines Promotor-Luciferase-Konstruktes wird aus dem Vektor pCLN (pCaMVLN; Callis et al. 1987) die BamHI/HindIII Kassette herausgeschnitten. Sie enthält das Luciferase Strukturgen (LUC) aus *Photinus pyralis* mit der Polyadenylierungsregion des Nopalin Synthase Genes. Diese Kassette wird in BamHI/HindIII geschnittenen Vektor pBluescript II SK (Stratagene) kloniert. Das Promotor/Leader-Fragment des Genes der Untereinheit c (Isoform 2) der V-Typ H⁺-ATPase aus *Beta vulgaris* wird aus dem Konstrukt pBVA/16-2 GUS (s.o.) mit BamHI herausgeschnitten. Der Vektor pBluescript II SK mit der einklonierten Luciferase-Kassette wird ebenfalls mit BamHI aufgeschnitten und der Promotor so "sticky-end" vor die Luciferase kloniert. Durch verschiedene Restriktionsverdaue (z.B. BamHI, SalI oder XmnI) wird geprüft, daß sich das Promotorfragment in dem Konstrukt in der richtigen Orientierung (5'->3') vor dem Luciferase Gen befindet. Die Übergänge von der MCS des Vektors zum Promotor und von der anderen Seite der MCS in die Luciferase werden zur Kontrolle mit den Vektorprimern "M 13 Forward" und "M 13 Reverse" sequenziert. Das Promotor/Leader-LUC-Konstrukt wird als pBVA/16-2 LUC bezeichnet.

### III. Methoden zur Messung der Promotoraktivitäten in Beta-Zellkulturen

### Ballistische Transformation

Die Methode der ballistischen Transformation (engl.: particle gun bombardment) wurde zur transienten Expression von Promotorgenkonstrukten in *Beta vulgaris* Zellsuspensionskulturen eingesetzt. Es handelt sich um eine Methode des direkten Gentransfers, wobei die DNA durch mechanische Zerstörung der Zellwand in die pflanzliche Zelle gelangt (Sanford et al. (1987)).
Es wurde ein Biolistic PDS-1000/He, Particle Delivery System, BIORAD verwendet.

Während des Versuchsablaufs kommt es durch eine Erhöhung des Gasdrucks in der Überdruckkammer zur Zerstörung der Berstscheiben. Die Anzahl der verwendeten Berstscheiben reguliert hierbei den in der Überdruckkammer erzeugten Druck. Folge ist ein schockartiges Entweichen des Heliumgasstromes aus der Überdruckkammer in die zuvor evakuierte Unterdruckkammer. Der entweichende Gasstrom beschleunigt den mit Partikeln beschichteten Makrocarrier, welcher bereits nach wenigen Zentimetern durch ein Fangnetz abgebremst wird. Die mit DNA beladenen Mikrocarrier (hier Wolframpartikel) passieren hingegen die Poren des Netzes und durchschlagen beim Auftreffen auf das Pflanzenmaterial die Zellwände.

### Mikrocarrierpräparation

30 mg Wolframpartikel (Mikrocarrier) werden in einem Reaktionsgefäß nach Zugabe von 1 ml 70 %igem Ethanol für 20 sec gevortext. Eine anschließende 10 minütige Inkubation ermöglicht ein Absetzen der Partikel, welche hierauf für 30 sec bei 1300 g (ungebremst) und Raumtemperatur sedimentiert werden. Nach Entfernung des Überstandes werden die Partikel mit 500 ml sterilem H20-Bidest wie folgt gewaschen: 10 Sekunden vortexen, 10 Minuten stehenlassen, 30 Sekunden ungebremste Zentrifugation bei 1300 g. Hierauf werden die sedimentierten Partikel mit 500 µl 50 %igem Glycerin versetzt und durch Vortexen gemischt. Die Lagerung erfolgt bei -20 °C.

### Beladen der Mikrocarrier mit DNA

Die für die Transformation benötigte Plasmid DNA soll frei von Verunreinigungen (Protein, RNA) sein und überwiegend in der "supercoiled" Form vorliegen. Zur Isolierung der DNA werden kommerziell erhältliche "Plasmid-Kits" eingesetzt. Für die Beladung der Partikel (Mikrocarrier) empfiehlt es sich jeweils eine Mindestanzahl von 10-15 Schuß vorauszusetzen. Während der Präparation kommt es schnell zu einem Absetzen der Mikrocarrier in der Suspension, kleine Volumina erschweren hierbei das Resuspendieren. Die nachfolgenden Volumenangaben beziehen sich jeweils auf 1 Schuß.
Alle Arbeitsgänge, die Zentrifugationsschritte ausgenommen, werden in der Sterilwerkbank durchgeführt. Die Partikelsuspension wird auf einem Vortex gründlich durchmischt und nachfolgend je 9 µl der Lösung in ein Reaktionsgefäß überführt. Unter stetigem Vortexen erfolgt die Zugabe von 1 µl Plasmid DNA (1 µg/µl in TE-Puffer). Das Gemisch wird für 15 Minuten in einem Eisbad gelagert. Daraufhin werden nacheinander 9 µl 2,5 M CaCl₂ (steril), 3,6 µl 1,2 mM Spermidin (steril filtriert) sowie 18,2 µl absoluter Ethanol unter leichtem Vortexen hinzupipettiert. Die Partikel werden im Anschluß an eine 10 minütige Inkubation im Eisbad für 5 Sekunden bei 180 g und Raumtemperatur sedimentiert, der Überstand wird hierauf entfernt. Nach Zugabe von 6,4 µl absolutem Ethanol werden die Partikel durch "Fingervortexen" vorsichtig resuspendiert und bis zur weiteren Verwendung im Eisbad gelagert.

### Vorbereitung des Zellmaterials

Für den Gentransfer wird die verwendete Zellsuspensionskultur (hier 3-4 Tage alte *Beta vulgaris* Kultur) auf Agarböden transferiert. Zur Steigerung der Transformationseffizienz wird der osmotische Wert des Mediums durch Zusatz von Mannitol und Sorbitol erhöht und die Zellen für ca. 4 Stunden hierauf präinkubiert. Der Einfluß der osmotischen Behandlung auf die Transformationseffizienz der Pflanzenzellen läßt sich vermutlich durch die eintretende Plasmolyse erklären (Vain et al., 1993). Hierdurch wird unter Umständen ein Ausfließen des Protoplasmas, nach Eindringen des Partikels in die Zeile, verhindert (Armaleo et al., 1990; Sanford et al., 1992).

Alle Arbeitsgänge, die Zentrifugationsschritte ausgenommen, erfolgen in der Sterilwerkbank. Für die Durchführung werden 10 ml Einwegpipetten, Pinzetten, Rundfilter (45 mm ∅), Büchnertrichter und eine Waschflasche bereitgestellt. Die Materialien, ausgenommen Einwegpipetten und Pinzetten, werden zuvor autoklaviert. Des weiteren werden Petrischalen (50 mm ∅) mit Gamborg B5 Medium (0,9 % Agar, supplementiert mit 0,5 g/l Caseinhydrolysat, 125 mM Sorbitol, 125 mM Mannitol nach Ingersoll et al., 1996) verwendet.
Ein Rundfilter wird auf dem Büchnertrichter aufgelegt. Anschließend werden 3,3 ml Suspensionskultur, entsprechen ca. 1 ml gepacktem Zellvolumen (s.u.), mittels Einwegpipette gleichmäßig auf der Filteroberfläche verteilt. Das überschüssige Medium wird durch kurzzeitiges Anlegen des Vakuums (ca. 2 Sekunden) über die Waschflasche abgesaugt. Der Filter wird auf eine Petrischale mit Nährmedium überführt, und die so vorbereiteten Zellen werden bis zum Gentransfer für 3-4 Stunden bei Raumtemperatur in der Sterilwerkbank gelagert.

### Bestimmung des 'CPV'

Zu Beginn des Versuches wird das gepackte Zellvolumen, CPV (engl.: cell packed volume) der Zellsuspensionskultur bestimmt. Hierfür werden 10 ml Suspension steril entnommen und in einem 15 ml skalierten Zentrifugenröhrchen für 5 Minuten bei 1130 g (Ausschwingrotor) sedimentiert. Das gepackte Zellvolumen wird anhand der Skalierung bestimmt.

### Transformation

Alle Arbeitsschritte, ausgenommen der Ultraschall-Behandiung der Partikelsuspension (s.u.), erfolgen unter der Sterilwerkbank. Zuvor werden das Innere der Partikelkanone sowie alle Metall- und Kunststofftäger mit absolutem Ethanol gereinigt. Des gleichen werden die Fangnetze und Berstscheiben mit Ethanol behandelt und unter der Sterilwerkbank getrocknet. Die Makrocarrier (M-20, BIORAD) werden durch kurzes Eintauchen in absolutem Ethanol sterilisiert und in einen Exsikkator überführt.
Die Wolframpartikeisuspension wird für 2 Sekunden bei maximaler Einstellung sonifiziert (Sonopuls HD 60, Bandelin) und je 5 µl der Suspension (ca. 422 µg Partikel 0,78 µg DNA) vorsichtig auf die Oberfläche eines Makrocarriers pipettiert. Um ein Absetzen zu verhindern, werden die Partikel zwischen den Pipettierschritten wiederholt durch Fingervortexen des Reaktionsgefäßes in Lösung gehalten. Der Exsikkator wird hierauf für 20 Minuten evakuiert. Vor dem Beschuß werden die Berstscheiben (3 Stück), die Partikel-beladenen Makrocarrier sowie das Fangnetz entsprechend der Anleitung befestigt. Für den mit der Suspensionskultur beschickten Petrischalenhalter wird die Position 3 gewählt Anschließend wird die Kammer bis zu einem Vakuum von 27inches (Hg) evakuiert und hierauf das Heliumgas in die Überdruckkammer eingeleitet. Bei einem Druck von 1.200 psi kommt es zum Zerstören der Berstscheiben. Im Anschluß werden die Petrischalen steril verschlossen (Parafilm) und die Kulturen für 2 Tage bei 24 °C im Dauerdunkel inkubiert.

### Histochemischer Nachweis der β-Glucuronidase Aktivität

Die transiente Expression des β-Glucuronidase Strukturgens kann durch ein histochemisches Verfahren nachgewiesen werden. Hierfür werden, im Anschluß an die Transformation und die Inkubationszeit von 24 h, Filterpapier und Suspensionkulturen in eine Petrischale (50 mm ∅) überführt und mit 1 ml Reaktionspuffer (s.u.) überschichtet. Die Inkubation erfolgt über Nacht bei 37 °C im Inkubationsschrank. Die Anzahl blau gefärbter Bereiche wird mittels Binokular bei einer 40fachen Vergrößerung bestimmt.
Zur Herstellung des Reaktionspuffers werden 70 mg X-Gluc (5-Bromo-4-Chloro-3-Indolyl β-D-Glucuronsäure, Duchefa) in 500 µl Dimethylsulfoxid vorgelöst und in 100 ml einer Pufferlösung (115 mM NaH₂PO₄ x 2 H₂0/ Na₂HPO₄ x 2 H₂0 pH 7, 10 mM Na₂-EDTA, 0,5 mM K₃[Fe(CN)₆], 0,5 mM K₄[Fe(CN)₆]) resuspendiert. Aliquots des so gewonnenen Reaktionspuffers können für mehrere Wochen bei - 20 °C aufbewahrt werden.

### Methoden zur Quantifizierung der β-Glucuronidase und Luciferase-Aktivitäten

Zur Quantifizierung der Enzymaktivität der verwendeten Reportergenprodukte wurden biolumineszente sowie chemilumineszente Nachweisverfahren eingesetzt. Zur Bestimmung der Enzymaktivität werden die Proteine zuvor extrahiert. Die in den anschließenden enzymatischen Reaktionen erzeugte "Lichtmenge" wird in einem Lumineszenz Spektrometer (Berthold, Lumat 9501) quantifiziert. Der erhaltene Meßwert repräsentiert die über einen Zeitraum integrierte Lichtmenge.
Die Angabe erfolgt als gemessene Lichteinheiten, "LU" (engl.: light unit).

### Herstellung der Proteinextrakte

Die Extraktion der Proteine erfolgte unter Verwendung des "GUS-Light Assay" (Tropix) sowie des "Luciferase Assay System" (Promega).

Zur Herstellung der Proteinextrakte werden die Suspensionskulturen mittels Spatel vom Filterpapier entfernt und, nach Bestimmung des Frischgewichtes, unter Zusatz von flüssigem Stickstoff im Mörser aufgeschlossen. Dem gefrorenen Material wird Extraktionspuffer (500 µl/g FG) hinzupipettiert und der Extrakt nach Auftauen gründlich homogenisiert. Die Suspension wird unter Verwendung abgeschnittener Pipettenspitzen in ein auf Eis gelagertes Reaktionsgefäß überführt und für 1 Minute bei 4 °C und 16.000 g zentrifugiert. Die Überstände werden bis zur "Aktivitätsbestimmung" auf Eis aufbewahrt.

### Extraktionspuffer: ("GUS-Light Assay")

Der Pufferlösung (GUS Lysis Solution) wird vor der Verwendung β-Mercaptoethanol hinzugesetzt (Endkonz.: 50 mM Na-Phosphat Puffer pH 7, 10 mM Na₂-EDTA, 0,1 % SDS, 0,1 % Triton, 10 mM β-Mercaptoethanol). Die Lösung wird bis zur Verwendung bei Raumtemperatur gelagert.

### Extraktionspuffer: (Luciferase Assay System)

Der 5x konzentrierte Puffer (Cell Culture Lysis Reagent) wird mit sterilem H20-Bidest 1:5 verdünnt (Endkonz.: 25 mM Tris-HCl pH 7,8 mit H₃PO₄, 2 mM CDTA, 2 mM DTT, 10 % Glyzerin, 1 % Triton) und bis zur Verwendung bei Raumtemperatur gelagert.

### β-Glucuronidase-Aktivitätsbestimmung

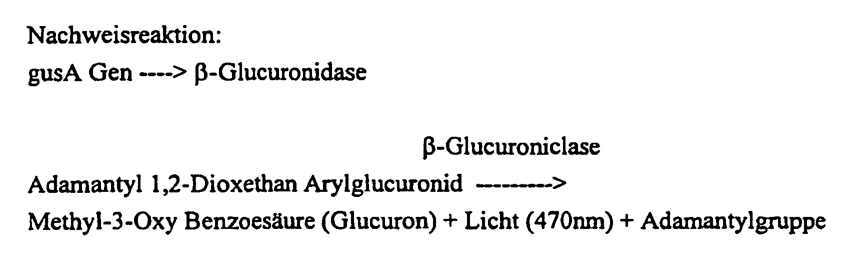

Zum Nachweis der β-Glucuronidase-Aktivität wurde der GUS-Light Kit (Tropix) verwendet. Die Durchführung erfolgte nach Angaben des Herstellers.
Proteinextrakt, Reaktionspuffer sowie "Accelerator" werden vor Verwendung auf Raumtemperatur vorgewärmt. Nach der Überführung von je 20 µl Proteinextrakt in ein Teströhrchen (10 ml) werden die Reaktionen durch zeitversetzte (20 Sekunden) Zugabe von Reaktionspuffer je 180 µl) gestartet. Die Inkubation erfolgt für 1 Stunde bei Raumtemperatur im Dauerdunkel. Anschließend werden den Reaktionen je 300 µl Accelerator-Lösung hinzupipettiert (zeitversetzt s.o.). Die einzelnen Proben werden unmittelbar nach Zugabe der Lösung im Lumineszenz Spektrometer (Lumat 9501, Berthold) mit 5 Sekunden Verzögerung über einen Zeitraum von ebenfalls 5 Sekunden vermessen.

### Negative Kontrolle

Die in dem Luminezenz Spektrometer ermittelten Absolutwerte lassen sich nicht unmittelbar auf eine Fremdgenaktivität zurückführen. Zur Bestimmung des Hintergrundwertes, der sich im Falle der β-Glucuronidase u.U. auch auf eine vergleichbare endogen vorliegende Aktivität zurückführen läßt, werden nichttransformierte Zellsuspensionskulturen mittels der zuvor beschriebenen Nachweisverfahren untersucht. Die Meßwerte dieser negativen Kontrolle (BW, Blindwert; siehe Tabelle 1a und 1b) erfassen darüber hinaus die durch Puffersubstanzen bedingten Hintergrundwerte. Für die Berechnung der Promotoraktivitäten wird der Blindwert von den Meßwerten der transformierten Kultur subtrahiert.

### Reaktionspuffer ("GUS Reaction Buffer")

Das Substrat (Glucuron, Chemiluminescent Substrate) wird vor der Verwendung 1:100 in "GUS Reaction Buffer" (nichtkomplementiert: 0,1 M Na-PhosphatPuffer, pH 7, 10 mM Na₂-EDTA) verdünnt.

### Luciferase-Aktivitätsbestimmung

### Nachweisreaktion:

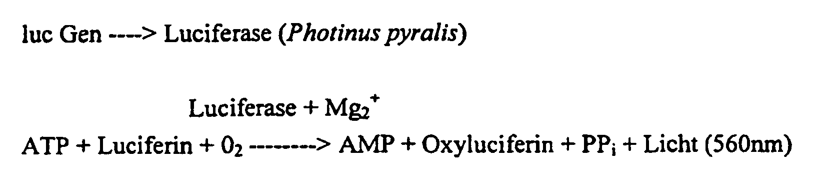

Zur Bestimmung der Luciferase-Aktivität wurde das Luciferase Assay System (Promega) verwendet. Das Verhältnis Proteinextrakt zu Reaktionspuffer wurde hierbei variiert.
Proteinextrakt sowie Reaktionspuffer s.u. werden auf Raumtemperatur vorgewärmt. Nach der Überführung von je 20 µl (100 µl) Proteinextrakt in ein 10 ml Teströhrchen, werden der Probe 100 µl (50 µl) Reaktionspuffer hinzugefügt, und der Ansatz wird hierauf unmittelbar im Lumineszenz Spektrometer (Lumat 9501, Berthold) über einen Zeitraum von 10 Sekunden vermessen. Entsprechend den Angaben zur Bestimmung der β-Glucuronidase-Aktivität wird auch hier ein Blindwert ermittelt, welcher zur Korrektur der Meßwerte transformierter Kulturen herangezogen wird.

### Reaktionspuffer ("Luciferase Assay Reagent")

Zur Herstellung des Reaktionspuffers wird das Substrat (Luciferase Assay Substrate) im "Luciferase Assay Buffer" gelöst (Endkonzentration: 470 µM Luciferin, 270 µM CoenzymA (Lithiumsalz), 530 µM ATP, 20 mM Trizin, 1,07 mM (MgCO₃)4Mg(OH)₂ x 5H₂0, 2,67 mM MgSO₄, 0,1 mM Na₂-EDTA, 33,3 mM DTT, pH 7,8). Der Reaktionspuffer wird aliquotiert und bei -70 °C gelagert.

### Bestimmung der relativen Promotoraktivität

Zur Quantifizierung der Promotoraktivität in Promotor/Reportergen-Konstrukten wird die transiente Expression der Reportergene mittels der oben beschriebenen enzymatischen Nachweisverfahren erfaßt. Bei der Verwendung des direkten Gentransfers zur Bestimmung der Promotoraktivität ergibt sich jedoch zwischen den einzelnen Transformationen oftmals eine weite, Streuung der gewonnenen Meßwerte. Im Fall der ballistischen Transformation wird die Effizienz des Gentransfers zum einen durch den physiologischen Zustand des transformierten Gewebes nach dem Beschuß beeinflußt. Zum anderen sind physikalische Faktoren von entsprechender Bedeutung, so z.B. Unterschiede in der Beladung der Mikrocarrier bzw. der Verteilung der Partikelsuspension auf dem Makrocarrier. Die Absicherung der Meßwerte erfordert daher eine Kalibrierung des Meßsystems.

### Theorie der Kalibrierung

### Testplasmid

Testpromotor: Zur Quantifizierung der Promotoraktivität wird die transiente Expression des Reportergens durch Bestimmung der enzymatischen Aktivität seines Genproduktes ermittelt. Die Promotoraktivität des Testpromotors kann jedoch nicht als Absolutwert der enzymatischen Aktivitätsbestimmung dargestellt werden. Die Quantifizierung erfordert den Vergleich mit einem konstitutiven Promotor (Standardpromotor).

Standardpromotor: In einem Parallelansatz wird die Expression des Reportergens unter einem konstitutiven Promotor (hier CaMV 35S Promotor) untersucht. Die Promotoraktivität des Testpromotors wird als Verhältnis: Aktivität_{Testpromotor}/Aktivität_{Standardpromotor} angegeben, wobei die Aktivität des Standardpromotors, gleich 1 gesetzt wird. Der Bezug auf die Expression des Reportergens unter einem Standardpromotor ermöglicht unter anderem den Vergleich der Ergebnisse aus Wiederholungsexperimenten.

### Kalibrierungsolasmid (interner Standard)

Zur Erfassung der Transformationseffizienz einzelner Beschüsse (Transformationen) werden die Mikrocarrier gleichzeitig mit einem Test- und einem Kalibrierungsplasmid beladen. Die verwendeten Konstrukte (Testplasmid, Kalibrierungsplasmid) tragen unterschiedliche Reportergene, wodurch eine parallele Erfassung ihrer Expressionsdaten (Enzymaktivitäten) ermöglicht wird. Das Strukturgen des Kalibrierungsplasmids steht unter der Kontrolle eines konstitutiven Promotors (hier CaMV d35S, verstärkter 35S Promotor). Die Meßwerte (Enzymaktivitäten) für das Kalibrierungsplasmid reflektieren die Transformationseffizienzen einzelner Beschüsse. Alle in einem Versuch erzielten Meßwerte für den internen Standard (Kalibrierungsplasmid) werden gemeinsam betrachtet und durch den im Versuch erzielten Maximalwert dividiert. Für den höchsten Meßwert ergibt sich folglich eine Transformationseffizienz von 1 (entspricht 100%). Die Transformationseffizienz (von 0-1) wird als relative Lichteinheit "rLU" (engl.: relative light units) angegeben. Die in der Untersuchung erzielten absoluten Meßwerte für das Testplasmid (Testpromotor, Standard-Promotor) werden unter Berücksichtigung der Transformationseffizienz des Beschusses korrigiert. Hierfür wird die für die Testplasmide ermittelte Aktivität durch die Transformationseffizienz dividiert. Die korrigierten Werte werden als relative Aktivitäten bezeichnet. Das Verhältnis Testplasmid zu Kalibrierungsplasmid (7:3, w/w) wird in allen Versuchen beibehalten.

### Kopienzahl

In der Transformation werden identische Mengen an Testplasmid-DNA eingesetzt (hier pro Beschuß: ca. 0,7 µg). Bei einem Vergleich der Aktivitäten von Testpromotoren und Standardpromotor (s.o.) muß daher die Plasmidgröße d.h. die Molekülanzahl (Kopiefaktor) pro pg Testplasmid-DNA berücksichtigt werden. Die pro Beschuß eingesetzte Molekülanzahl an Standardpromotor (Testplasmid) wird hierbei als Referenzwert betrachtet und gleich 1 gesetzt. Die Meßwerte für die Aktivität der Testpromotoren werden unter Berücksichtigung der Molekülanzahl (pro Beschuß) korrigiert.

### Berechnung der relativen Promotoraktivität

Zur weiteren Veranschaulichung zeigt das nachfolgende Beispiel eine exemplarische Darstellung der einzelnen Rechenschritte.
Die Angaben beziehen sich auf eine Untersuchung zur Quantifizierung der relativen Promotoraktivität des Promotors der A-Untereinheit (70 kDa) der V-Typ H⁺-ATPase aus *Beta vulgaris* (Testpromotor, Plasmid: pBVA-70/LUC). Als Bezugsgröße (Standardpromotor) diente der CaMV 35S Promotor (Plasmid: PCaMVLN). Beide Testplasmide tragen das Luciferase Strukturgen (LUC). Zur Erfassung der Transformationseffizienz wurde in der Kotransformation das Kalibrierungsplasmid pFF19G verwendet. Es trägt das β-Glucuronidase Strukturgen (GUS) unter der Kontrolle des verstärkten 35S CaMV Promotors.

Es wurden die folgenden Kombinationen von Testplasmid x Kalibrierungsplasmid eingesetzt:
1. Testpromotor: 70kDa UE-ATPase (pBVA-70/LUC) x CaMV d35S (pFF19G)
2. Standardpromotor: CamV 35S (PCaMVLN) x CaMV d35S (pFF19G)

**Tabelle 1a:**

| Berechnung der relativen Luciferase-Aktivität für das Promotorkonstrukt der 70 kDa Untereinheit der V-Typ H⁺-ATPase aus *Beta vulgaris* (70-kDa UE ATPase/LUC x CaMV d35S/GUS) | | | | | | | |
|---|---|---|---|---|---|---|---|
| Platte | LUC-Aktivität | LUC-Aktivität (-BW) | GUS-Aktivität | GUS-Aktivität (-BW) | Effizienz | relative LUC-Aktivität | Mittelwert |
| | LU | LU | LU | LU | rLU | rLU | rLU |
| 1 | 8070 | 7967 | 28734 | 22286 | 0,65 | 12257 | |
| 2 | 10977 | 10874 | 40238 | 33790 | 0,99 | 10984 | |
| 3 | 13834 | 13731 | 40640 | 34192 | 1 | 13731 | 12324 |

Hintergrundaktivität: Die Absolutwerte aus der Enzymaktivitätsbestimmung (LUC- und β-GUS-Aktivitat) transformierter Zellen wurde unter Berücksichtigung der Meßwerte der Negativkontrolle korrigiert (Absolutwert - Blindwert (BW)). Der Blindwert für die LUC-Aktivität betrug 103 LU. Der Blindwert für die GUS-Aktivität betrug 6448 LU. Die Angaben in LU (Lichteinheiten) beziehen sich auf die eingesetzte Menge an Proteinextrakt (hier 20 µl). Transformationseffizienz: In Platte 3 wurde der höchste Meßwert (GUS-Aktivität) für den internen Standard CaMV d35S/GUS und somit die höchste Transformationseffzienz erzielt. Die Werte für die LUC-Aktivitäten der restlichen Transformationsansätze wurden unter Berücksichtigung der jeweiligen Transformationseffizienz korrigiert.

**Tabelle 1b:**

| Berechnung der relativen Luciferase-Aktivität für den CaMV 35S Promotor (35S CamV/ LUC x CaMV d35S/GUS); siehe Erklärung zu Tabelle 1a | | | | | | | |
|---|---|---|---|---|---|---|---|
| Platte | LUC-Aktivität | LUC-Aktivität (-BW) | GUS-Aktivität | GUS-Aktivi-tät (-BW) | Effizienz | LUC-Aktivität (korr.) | Mittelwert |
| | LU | LU | LU | LU | rLU | rLU | rLU |
| 1 | 4518 | 4415 | 13756 | 7308 | 0,21 | 21024 | |
| 2 | 13572 | 13469 | 20566 | 14118 | 0,41 | 32851 | |
| 3 | 11256 | 11153 | 17051 | 10603 | 0,31 | 35977 | 29951 |

Zur Berechnung der Aktivität des Testpromotors wird der Mittelwert der relativen Luciferase-Aktivität (siehe Tabelle 1a) unter Berücksichtigung der Molekülanzahl (Kopiefaktor) korrigiert (s.u.). Die relative Stärke des Testpromotors wird nunmehr als das Verhältnis: Aktivität des Testpromotors/Aktivität des Standardpromotors ausgedrückt. Im Beispiel ergibt sich ein Wert von 0,49 für die relative Promotoraktivitit des Promotors der A-Untereinheit der V-Typ H⁺-ATPase aus *Beta vulgaris*.

| Promotor | LUCAktivität (rLU) | Kopiefaktor | LUC-Aktivität korrig. (rLU) | Promotorstärke |
|---|---|---|---|---|
| 70 kDa UE ATPase | 12324 | 1,2 | 14789 | 0,49 |
| 35S CaMV | 29951 | 1 | 29951 | 1 |

### IV. Aufbau der Plasmidkonstrukte für die ballistische Transformation

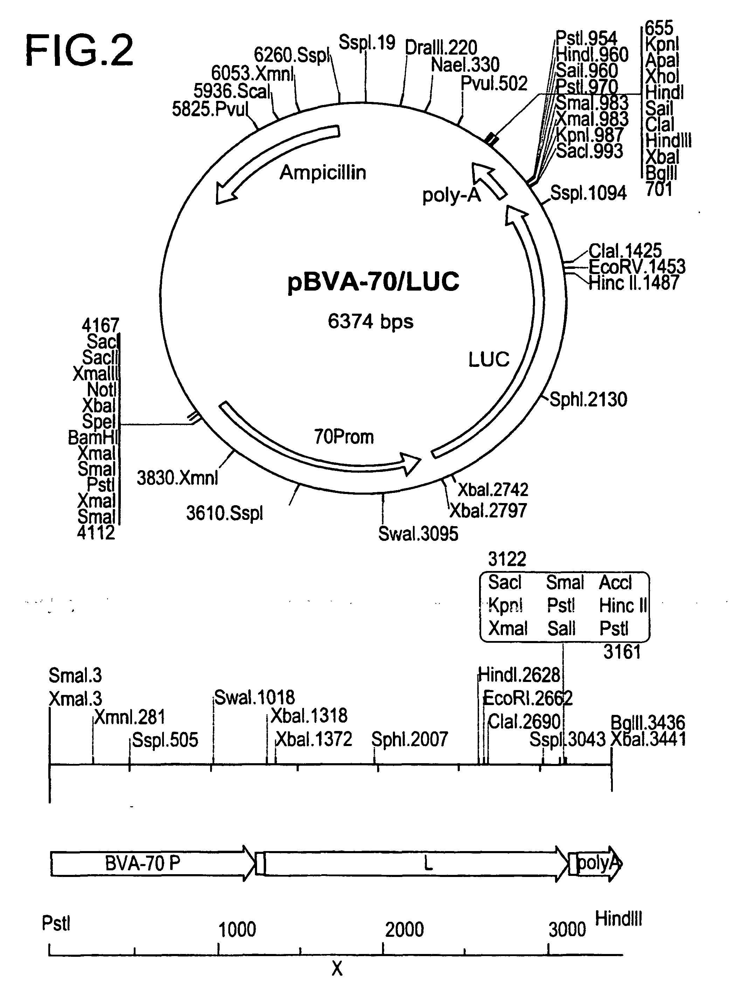

Figur 2 zeigt den Aufbau des Konstrukts pBVA-70/LUC. Zur Gewinnung des Luciferase-Konstrukts pBVA-70/LUC wurde der 5'-regulative Bereich (1236 bp) des Genes für die A-Untereinheit der V-Typ H⁺-ATPase aus *B. vulgaris* über einen PstI/BglII Verdau aus dem Plasmid pBVA-70 freigesetzt. Das genomische Fragment wurde anschließend vor die BamHI/HindIII Kassette des Vektors pCaMVLN, über die Schnittstellen PstI/HindIII in den pBlueskript SKII+ Vektor (Stratagene) kloniert. Die Kassette enthält neben dem Luciferase Strukturgen die Polyadenylierungsregion des Nopalin Synthasegens. Vektoranteile (■) sind in der Darstellung der BVA-70 Promotor-LUC-CaMV-Terminator Kassette besonders hervorgehoben.

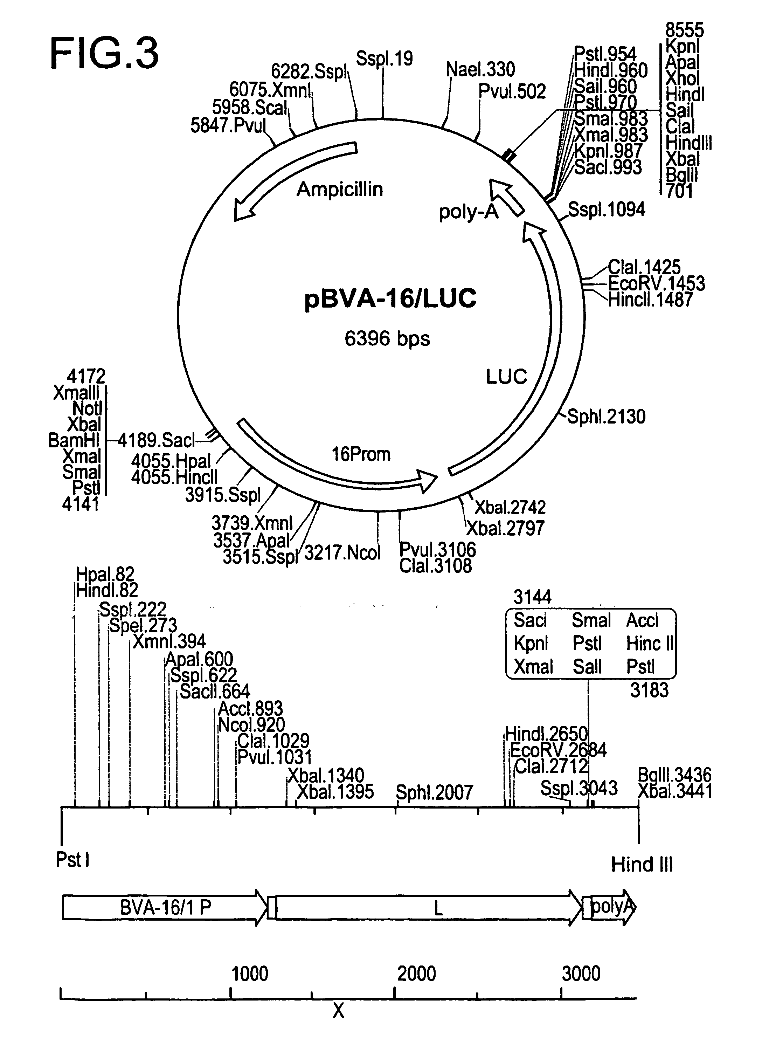

Figur 3 zeigt den Aufbau des Konstrukts pBVA-16/LUC. Zur Gewinnung des Luciferase-Konstrukts pBVA-16/LUC wurde das in Figur 2 beschriebene PstI/BglII Fragment des genomischen Subklon pBVA-16/1 vor die BamHI/HindIII Kassette des Vektors pCaMVLN, über die Schnittstellen PstI/HindIII in den pBlueskript II SK+ Vektor (Stratagene) kloniert. Siehe Erläuterungen zu Figur 2.

### V. Darstellung der klonierten 5'-Deletionen für die V-ATPase-Promotoren A, c1 und c2

### Promotor-Deletionen des BVA/16-2 Promotor-LUC-Konstruktes

Als Ausgangskonstrukt für die Promotor-Deletionen wird das Promotor/Leader-Luciferase-Konstrukt pBVA/16-2 LUC eingesetzt. Dieses Konstrukt enthält den gesamten Promotorbereich mit 1923 bp und zusätzlich 87 bp des Leader (-1923 bis + 87 = 2010 bp). Der ATPase Promotor soll mit Hilfe des "Exo Mung Bean Deletion Kit" (Stratagene, 1997) ausgehend vom 5'-Ende deletiert werden.

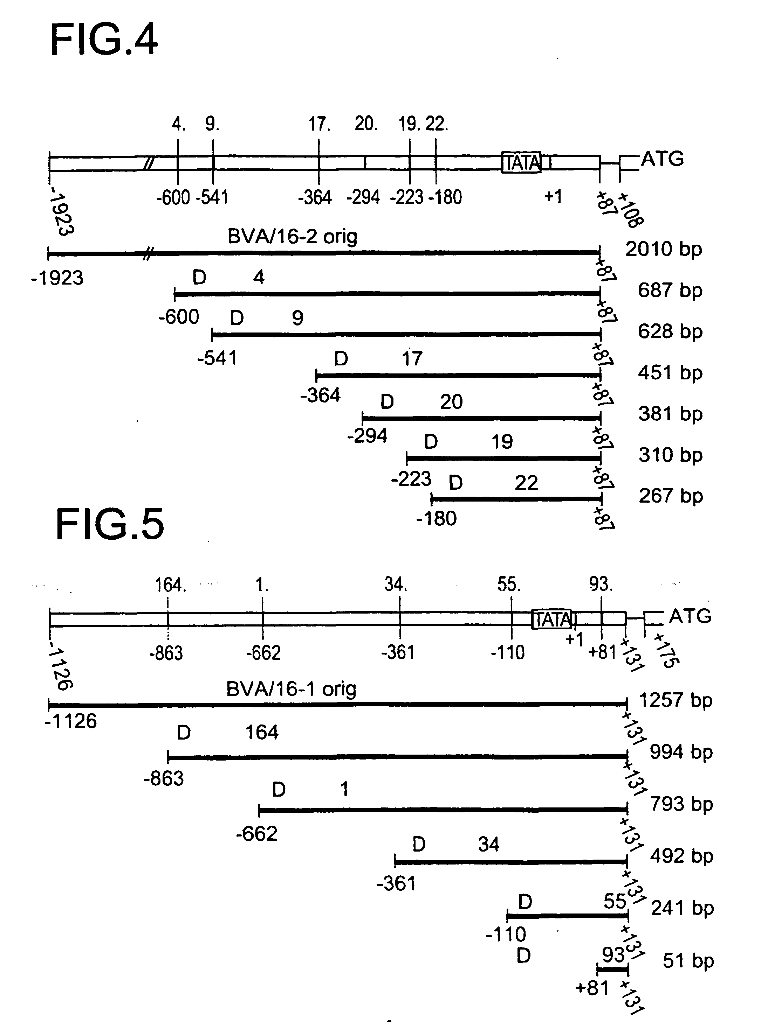

Figur 4 zeigt die BVA/16-2 Promotor-Deletionen. In der obersten Reihe der Abbildung ist die Promotorregion vom ursprünglichen genomischen Klon pBVA/16-2 mit dem Leader bis zum Translationsstart (ATG) gezeigt. Der Transkriptionsstart wurde mit +1 markiert. Darunter werden die einzelnen Deletionsklone schematisch dargestellt mit der Nummer des Deletionsklones. Die Zahl jeweils links zeigt die Länge des verbleibenden Promotors und die Zahl rechts am Ende jeder Reihe die Gesamtlänge von deletiertem Promotor und *Leader-Anteil* (je +87 bp) im Promotor-Luciferase-Konstrukt.

Damit der Vektor nicht auch mitverdaut wird, wird eine Restriktionsschnittstelle für ein Enzym in der MCS links des Vektors ausgesucht, das weder im Promotor noch im Reportergen schneidet. Bei einem Restriktionsenzym mit einem 3'-Überhang wird keine Fill-In Reaktion benötigt,. Bei einem Restriktionsenzym mit einem 5'-Überhang, z.B. NotI, wird nach dem Verdau das "sticky-end" mit Hilfe der Klenow-Polymerase mit α-Thio dNTPs aufgefüllt. Der Promotor wird zuerst 5'-seitig mit einem SpeI Verdau um 1121 bp vom 5-Ende her gekürzt. Im Promotor/Leader Fragment des Konstruktes bleiben 812 bp des Promotors plus 87 bp des Leaders = 889 bp. Durch den SpeI Verdau entsteht ein 5-Restriktionsüberhang. Das Enzym Exonuklease III verdaut 5'-Restriktionstüberhänge, die nicht mit (α-Thio dNTPs aufgefüllt wurden. Nach einem NotI Verdau des Konstruktes mit anschließendem Auffüllen mit α-Thio dNTPs und einem darauffolgenden SpeI Verdau kann die 5'-Deletion des Promotors mit der Exonuklease III gestartet werden (es werden zunächst 10 Deletionszeitpunkte gewählt). Das Vektorende des Konstruktes bleibt dabei unverändert. Nach dem Verdau mit der Exonuklease III werden die verbleibenden Überhänge mit Mung Bean Nuklease verdaut. Man erhält so zwei glatte Enden, die ligiert werden und anschließend in *E. coli* kompetente Zellen transformiert werden können. Nach Plasmid-Isolationen und anschließenden Testverdaus mit SacI/XbaI werden die ausgewählten Plasmide mit dem T3-Vektorprimer (Senseprimer) ansequenziert (Firma TOPLAB). Ausgehend von der MCS links wird so der Übergang vom Vektor pBluescript II SK in den 5'-deletierten Promotor sequenziert. In der ballistischen Transformation mit der Partikelkanone werden ausgesuchte Deletionsklone eingesetzt: Nr. 4 mit 600 bp Promotor (687 bp Promotor + Leader), Nr. 9 mit 541 bp Promotor (628 bp Promotor + Leader), Nr. 17 mit 364 bp Promotor (451 bp Promotor + Leader), Nr. 20 mit 294 bp Promotor (381 bp Promotor + Leader), Nr. 19 mit 223 bp Promotor (310 bp Promotor + Leader) und Nr. 22 mit 180 bp Promotor (267 bp Promotor + Leader).

### Promotor-Deletionen des BVA/16-1 Promotor-LUC-Konstruktes

In nachfolgenden Untersuchungen soll der Promotor der Isoform 2 der Untereinheit c der V-Typ H⁺-ATPase aus *B. vulgaris* (BVA/16-2) mit dem Promotor der weiteren bisher bekannten Isoform (Isoform 1 Untereinheit c der V-Typ H⁺-ATPase, *Beta vulgaris,* BVA/16-1) verglichen werden. Daher werden mit dem Promotor/Leader-Luciferase-Konstrukt pBVA/16-1 LUC (Lehr et al. 1999) ebenfalls 5'-Promotor-Deletionen durchgeführt (siehe oben). Dieser Promotor (BVA/16-1) wurde als PstI/Bgfil Fragment vor die BamHI/HindIII Kassette des Vektors pCLN (mit dem Luciferase Strukturgen) in den Vektor pBluescript II SK + kloniert. Das Ausgangskonstrukt enthielt den gesamten Promotorbereich mit 1126 bp und zusätzlich 131 bp des Leaders (-1126 bis + 131 = 1257 bp). Die Deletionsklone werden zum Abschluß ebenfalls ansequenziert. Als Kontrolle zu den Promotor Deletionsklonen wird ein Klon hergestellt. in dem bis in den Leader deletiert wird. Er enthält also nur noch 51 bp (+81 bis + 131) des Leaders vor der Luciferase. In der ballistischen Transformation mit der Partikelkanone werden zunächst ausgesuchte Deletionsklone eingesetzt Nr.164 mit 863 bp Promotor (994 bp Promotor + Leader), Nr. 1 mit 662 bp Promotor (793 bp Promotor + Leader), Nr.34 mit 361 bp Promotor (492 bp Promotor + Leader), Nr.55 mit 110 bp Promotor (241 bp Promotor + Leader) und der Kontrolle Nr.93 mit 51 bp des Leaders ohne Promotor.

Figur 5 zeigt die BVA/16-1 Promotor-Deletionen. In der Abbildung ist din der obersten Reihe die Promotorregion vom ursprünglichen genomischen Klon pBVA/16-1 mit dem Leader bis zum Translationsstart (ATG) gezeigt. Der Transkriptionsstart wurde mit +1 markiert. Darunter werden die einzelnen Deletionsklone schematisch dargestellt mit der jeweiligen Nummer des Deletionsklones. Die Zahl jeweils links zeigt die Länge des verbleibenden Promotors und die Zahl rechts am Ende jeder Reihe die Gesamtlänge von deletiertem Promotor und Leader-Anteil (je +131 bp) im Promotor-Reportergen-Konstrukt.

### Deletionen für den V-ATPase A-Promotor

Das Ausgangskonstrukt pBVA/70-LUC enthielt 1035 bp 5'-upstream Sequenz bezogen auf den Transkriptionsstart.
Folgende Deletionen wurden, analog zum Verfahren bei c1 und c2, hergestellt:

**Tabelle 2:**

| Deletionskonstrukte von pBVA/70-LUC | |
|---|---|
| Deletion | Position bezogen auf Transkriptionsstart |
| d 15/47 | -30 |
| d 15/7 | -108 |
| d 15/49 | -164 |
| d 13/14 | -270 |
| d 9/180 | -356 |
| d 6/80 | -494 |
| d 4/47 | -591 |
| d 4/46 | -682 |
| 70-5LUC (=pBVA-70-LUC) | -1035 |

### VI. Vergleich der Promotoraktivitäten von A, c1, c2 und CaMV35S unter Standardbedingungen

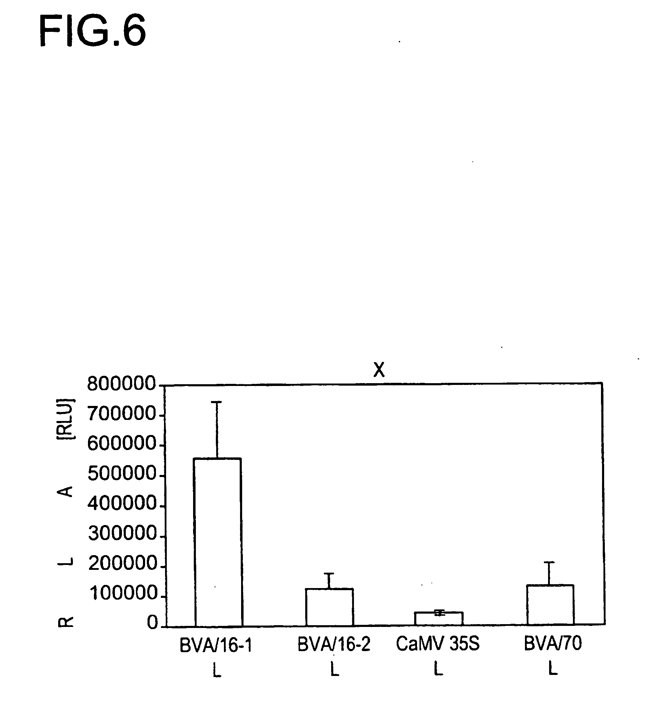

Figur 6 zeigt den Aktivitätsvergleich verschiedener Promotoren unter Kontrollbedingungen. In diesem Versuch der transienten Genexpression mittels der ballistischen Transformation werden die Aktivitäten der einzelnen Promotoren indirekt über die Luciferase-Aktivität bestimmt. Die drei V-Typ H⁺-ATPase Promotoren BVA/16-1, BVA/16-2 und BVA/70 werden mit dem CaMV 35S Promotor verglichen. Die Endwerte der Luciferase-Aktivitäten werden unter anderem über die Kotransformation mit dem Konstrukt pFF19G untereinander abgeglichen. Im Diagramm sind die korrigierten Endwerte mit den Standardabweichungen zu sehen.

**Tabelle 3:**

| Endwerte mit Promotorstärken (bezogen auf pCLN bzw. 16-2) | | | | |
|---|---|---|---|---|
| | | korrigierte Endwerte der Luciferase-Aktivitäten [RLU] | | |
| 1. | pBVA/16-1 LUC pFF19G (1:1) | 561355 | 14,06 | 4,64 |
| 2. | pBVA/16-2 LUC pFF19G (1:1) | 120958 | 3,03 | 1 |
| 3. | pCLN pFF19G (1:1) | 39912 | 1 | |
| 4. | pBVA/70 LUC pFF19G (1:1) | 134384 | 3,36 | |

Bei diesem Experiment unter Kontrollbedingungen ist der V-ATPase BVA/16-1 Promotor annähernd 5 x aktiver als der BVA/16-2 Promotor. Die Promotoren BVA/16-2 und BVA/70 zeigen vergleichbare Aktivitäten (BVA/70 zeigt eine leicht höhere Aktivität als BVA/16-2). Im Vergleich mit dem CaMV 35 S Promotor im Konstrukt pCLN sind die V-ATPase Promotoren BVA/16-2 und BVA/70 etwa 3 x aktiver und der stärkste dieser drei ATPase Promotoren, BVA/16-1, zeigt eine 14 x höhere Aktivität als pCLN.

### Nachweis der konstitutiven Aktivität der V-ATPase-Promotoren für die Isoformen c 1 und c2 in Blättern von Beta vulgaris

Die Aktivitäten der Promotor-Reportergen-Konstrukte für c2 (Vollängenpromotor) und c1 (5'-Deletion d164) wurden in vollexpandierten Blättern von Freilandpflanzen mittels *particle bombardment* bestimmt. Hierzu wurden je 2 Blattstanzlinge (∅ 5cm) übereinander in Petrischalen auf feuchtem Filterpapier mit der Particle Gun beschossen (1 Berstscheibe, 900 psi). Beschuß mit 0.5 µg Plasmid (V-ATPase-Promotor/pFF19G=7:3). Nach Beschuß wurden die Blattstanzlinge in Petrischalen auf Wasser flottierend für 24 Std. im Licht inkubiert.

**Tabelle 4:**

| Nachweis der konstitutiven Aktivität der V-ATPase-Promotoren für die Isoformen c1 und c2 in Blättern von *Beta vulgaris* | | |
|---|---|---|
| Promotor | Exp.no. | LUC-Aktivität |
| CaMV 35S | 1 | 2348 |
| | 2 | 829 |
| | 3 | 832 |
| c1 (d164) | | 565 |
| | | 527 |
| | | 534 |
| c2 | | 1691 |
| | | 1404 |
| | | 669 |

Die Ergebnisse in Tabelle 4 zeigen, daß die Promotoren c2 und c1(d164) selbst in vollexpandierten Blättern zum CaMV 35S vergleichbare Aktivitäten aufweisen. Parallel hierzu ergab die Northern Blot-Analyse, daß erwartungsgemäß die c2-Isoform, wie für c1 bereits gezeigt, ebenfalls konstitutiv in Wurzel, jungem und altem Blatt exprimiert wird. Salzbelastung der Pflanzen führt, wie bereits für c1 beschrieben (Lehr et al. 1999) zu einer Erhöhung der Transkriptmenge.

### Northern Blot-Analyse zur Expression der c2-Isoform in Beta vulgaris

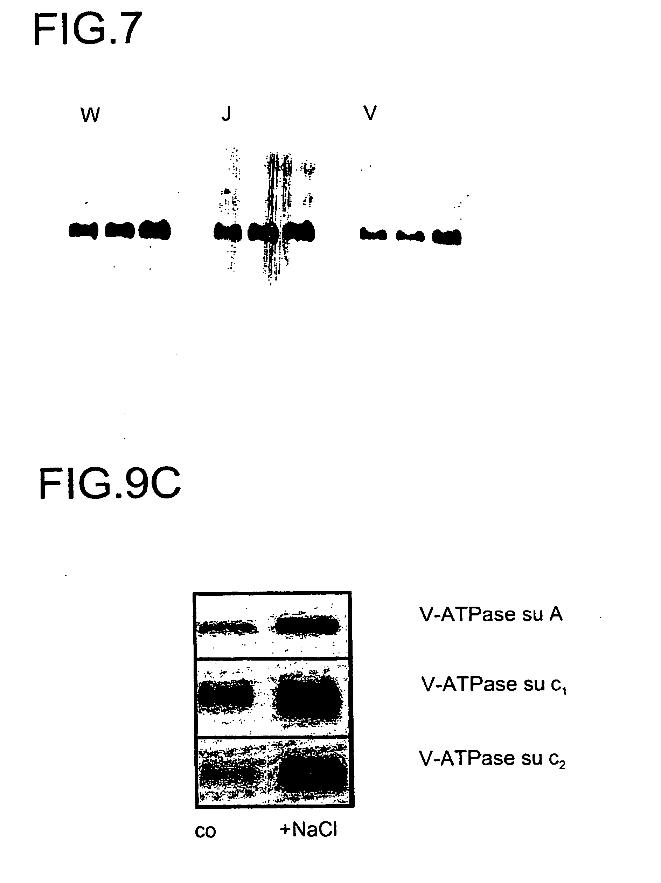

Figur 7 zeigt eine Northern Blot-Analyse zur Expression der c2-Isoform in *Beta vralgaris*. Es wurden jeweils 10 µg Gesamt-RNA aufgetragen und mit einer genspezifischen Sonde aus dem 3-UTR-Bereich hybridisiert. Es wurden jeweils zwei Proben aus Kontrollpflanzen aufgetragen (links, mitte) und eine Probe aus salzbehandelten (100mM, 48Std.) Pflanzen (rechts).

### VII. Relative Aktivitäten von 5'-Deletionen der Promotoren A, c1 und c2 im Vergleich zu CaMV35S

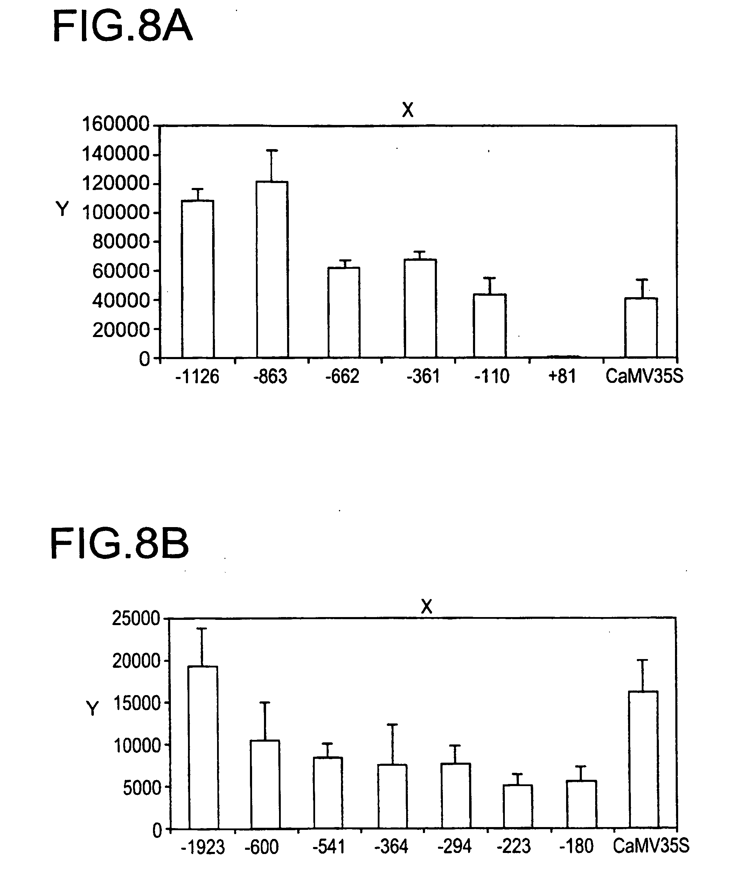

Figur 8 zeigt den Aktivitätsvergleich verschiedener deletierter Promotoren unter Kontrollbedingungen. In diesem Versuch der transienten Genexpression mittels der ballistischen Transformation werden die Aktivitäten der einzelnen Promotoren indirekt über die Luciferase-Aktivität bestimmt. Die die deletierten V-Typ H⁺-ATPase Promotoren von BVA/16-1 und BVA/16-2 werden mit dem CaMV 35S Promotor verglichen. Die Endwerte der Luciferase-Aktivitäten werden unter anderem über die Kotransformation mit dem Konstrukt pFF19G untereinander abgeglichen. Im Diagramm sind die korrigierten Endwerte mit den Standardabweichungen zu sehen. Die Nummern unter den Säulen bezeichnen die verschiedenen in Figur 4 und 5 dargestellten Deletionsfragmente.

### Promotoraktivitäten verschiedener 5'-Deletionen des V-ATPase A-Promotors im Vergleich zum CaMV 35S-Promotor

**Tabelle 5:**

| Aktivitätsvergleich verschiedener deletierter Promotoren des V-ATPase Promotors der Untereinheit A unter Kontrollbedingungen | |
|---|---|
| Promotor | LUC-Aktivität |
| CaMV 35S | 48766 |
| 70-5LUC (-1035) (Vollängen-Promotor) | 93459 |
| d4/46 (-682) | 90716 |
| d4/47(-591) | 89906 |
| d6/80 (-494) | 62572 |
| d9/180 (-356) | 56545 |
| d13/14 (-270) | 77619 |
| d15/49 (-164) | 43623 |
| d15/7 (-108) | 43046 |
| d15/47 (-30) | 44448 |

Tabelle 5 zeigt den Aktivitätsvergleich verschiedener deletierter Promotoren des V-ATPase Promotors der Untereinheit A unter Kontrollbedingungen. In diesem Versuch der transienten Genexpression mittels der ballistischen Transformation werden die Aktivitäten der einzelnen Promotoren indirekt über die Luciferase-Aktivität bestimmt. Die die deletierten V-Typ H⁺-ATPase Promotoren von BVA/70 werden mit dem CaMV 35S Promotor verglichen. Die Endwerte der Luciferase-Aktivitäten werden unter anderem über die Kotransformation mit dem Konstrukt pFF19G untereinander abgeglichen. Im Diagramm sind die korrigierten Endwerte mit zu sehen. Die Nummern der Promotoren bezeichnen die verschiedenen in Tabelle 3 genannten Deletionsfragmente.

### VIII. Promotoraktivitäten verschiedener 5'-Deletionen des V-ATPase c1-Promotors sowie des CaMV 35S-Promotors während des Wachstums einer Beta-Zellkultur

Tabelle 6 zeigt die Promotoraktivitäten verschiedener 5'-Deletionen des V-ATPase c1-Promotors sowie des CaMV 35S-Promotors während des Wachstums einer *Beta*-Zellkultur. Die Aktivitäten wurden in *Beta*-Suspensionszellen 1,5; 3,5; 5,5 sowie 7,5 Tage nach Umsetzen in frisches Medium bestimmt.

**Tabelle 6:**

| Promotoraktivitäten verschiedener 5'-Deletionen des V-ATPase c1-Promotors sowie des CaMV 35S-Promotors während des Wachstums einer *Beta-* Zellkultur | | | | |
|---|---|---|---|---|
| Tage nach Transfer | | Promotoraktivitäten (LUC) | | |
| | CaMV 35S | d5/55(-110) | d3/34(-361) | d0/164(-863) |
| 1,5 | 75141 | 51857 | 106816 | 165252 |
| 3.5 | 75170 | 59147 | 132474 | 219219 |
| 5.5 | 63916 | 44955 | 108072 | 157218 |
| 7.5 | 18192 | 16954 | 31877 | 51930 |

### IX. Einfluß von NaCl(KCl)-Stress auf die Promotoraktivitäten von A, c1, c2 und CaMV35S

### Einfluß von NaCl(KCl-Streß (125mM/48Std.) auf die Aktivitäten einer 5'-Deletion des A-Promotors (d4/46 [-682bp]) und einer 5'-Deletion des c1 - Promotors (d164 [-863] in Beta-Zellkulturen.

1,5 Tage nach dem letzten Transfer wurden die Zellen auf Rundfilter abgenutscht und diese auf Petrischalen mit Kontrollmedium bzw. unter Zusatz von 125 mM NaCl (KCl) inkubiert. Nach Beschuß erfolgte eine weitere Inkubation für 24 Std. Anschließend wurden die LUC-Aktivititen bestimmt.

**Tabelle 7:**

| Einfluß von NaCl- bzw. KCl-Streß auf die Aktivitäten der V-ATPase-Promotoren | | | |
|---|---|---|---|
| Promotor | Kontrolle | LUC-Aktivität | |
| | | +125mM NaCl | +125mM KCl |
| CaMV35S | 32527(100) | 8384(26) | 4711 (14) |
| d164(c1) | 67841 (100) | 51380(76) | 22379(33) |
| CaMV35S | 20924 (100) | 5036(24) | n.d. |
| d4/46(A) | 23165 (100) | 9886(43) | n.d. |

Die Resultate zeigen, daß die Aktivitäten der V-ATPase-Promotoren durch NaCl- bzw. KCl-Belastung der Zellen erheblich weniger beeinflußt werden als der CaMV35S-Promotor. Weitere Daten hierzu finden sich bei Lehr et al. (1999).

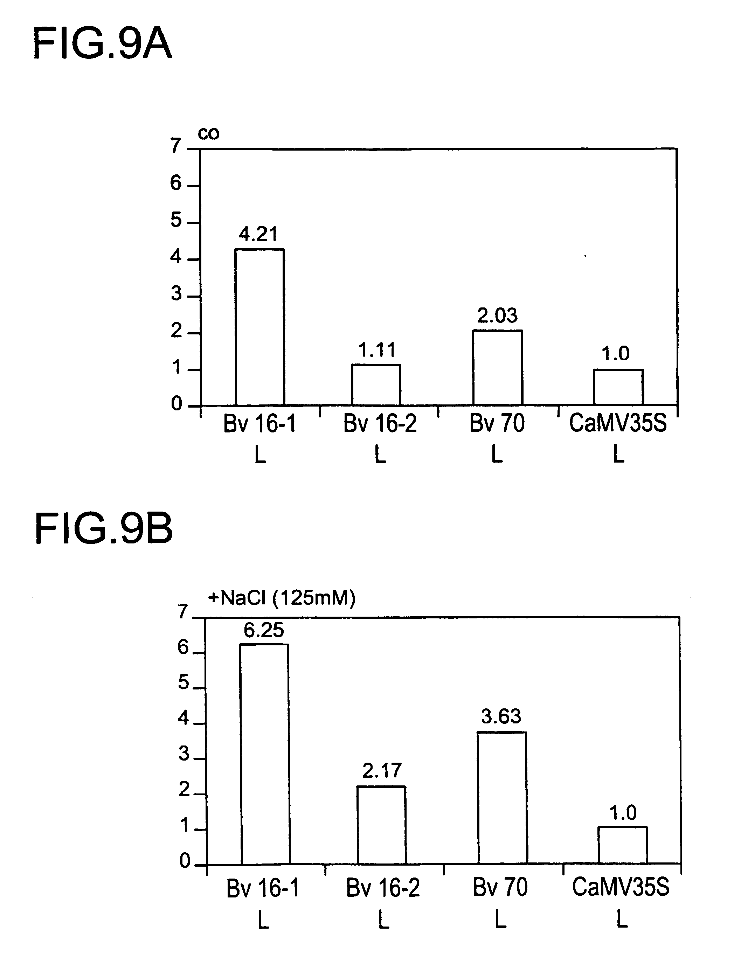

In Figur 9 wird gezeigt, daß ein vergleichbarer Effekt auch für den c2-Promotor beobachtet wird. Dargestellt sind die Aktivitäten nach Einfluß von 125 mM NaCl für mind. 24 Stunden. Zudem zeigt Figur 9 unten in der Northern Blot-Analyse, daß die Transkriptmengen für die V-ATPase-Gene A, c1 und c2 nach Salzexposition alle gegenüber der Kontrollbehandlung erhöht sind.

### X. Einfluß von Phosphat-Mangel auf die Promotoraktivitäten von A, c1, c2 und CaMV35S

### Einfluß von Phosphat-Mangel (48Std.) auf die Aktivitäten einer 5'-Deletion des A-Promotors (d4/46 [-682bp]) und einer 5'-Deletion des c1-Promotors (d164 [-8631]) in Beta-Zellkulturen

1,5 Tage nach dem letzten Transfer wurden die Zellen auf Rundfilter abgenutscht und diese auf Petrischalen mit Kontrollmedium bzw. Phosphat-freiem Medium für 48 Std. inkubiert. Nach Beschuß erfolgte eine weitere Inkubation für 24 Std. Anschließend wurden die LUC-Aktivitäten bestimmt.

**Tabelle 8:**

| Einfluß von Phosphat-Mangel (48Std.) auf die Aktivitäten einer 5'-Deletion des A-Promotors (d4/46 [-682bp]) und einer 5'-Deletion des c1-Promotors (d164 [-863]) in *Beta*-Zellkulturen | | |
|---|---|---|
| | LUC-Aktivität | |
| Promotor | Kontrolle (+P) | - Phosphat |
| CaMV35S | 33084 (100) | 6842 (20) |
| d164(c1) | 97417 (100) | 32897 (34) |
| CaMV35S | 20924 (100) | 2678 (13) |
| d4/46(A) | 23165 (100) | 7406 (32) |

Die Resultate zeigen, daß die Aktivitäten der V-ATPase-Promotoren durch Phosphat-Mangel der Zellen deutlich weniger beeinflußt werden als die Aktivität des CaMV35S-Promotors.

### XI. Einfluß von Saccharosemangel auf die Promotoraktivitäten von A, c1, c2 und CaMV35S

### Einfluß von Saccharosemangel (48Std.) auf die Aktivitäten einer 5'-Deletion des A-Promotors (d4/46 [-682bp]) und einer 5'-Deletion des c1-Promotors (d164 [-863]) in Beta-Zellkulturen

1,5 Tage nach dem letzten Transfer wurden die Zellen auf Rundfilter abgenutscht und diese auf Petrischalen mit Kontrollmedium bzw. Saccharose-freiem Medium für 48 Std. inkubiert. Nach Beschuß erfolgte eine weitere Inkubation für 24 Std. Anschließend wurden die LUC-Aktivitäten bestimmt.

**Tabelle 9:**

| Einfluß von Saccharose-Mangel (48Std.) auf die Aktivitäten einer 5'-Deletion des A-Promotors (d4/46 [-682bp]) und einer 5'-Deletion des c1-Promotors (d164 [-863]) in *Beta*-Zellkulturen | | |
|---|---|---|
| | LUC-Aktivität | |
| Promotor | Kontrolle (+S) | -Saccharose |
| CaMV35S | 33084 (100) | 31740 (95) |
| d164(c1) | 97417 (100) | 71017 (73) |
| CaMV35S | 20924 (100) | 6933 (33) |
| d4/46(A) | 23165 (100) | 6780 (29) |

Die Resultate zeigen, daß die Aktivitäten der V-ATPase-Promotoren durch Saccharosemangel der Zellen in ähnlichem Maße beeinflußt werden wie die Aktivität des CaMV35S-Promotors.

### XII. Koordinierte Wundinduktion der V-ATPase-Gene A, c1, c2 und E in Speichergewebe von Beta-Rüben

### Expression von V-ATPase- und V-PPᵢase-Genen nach Verwundung

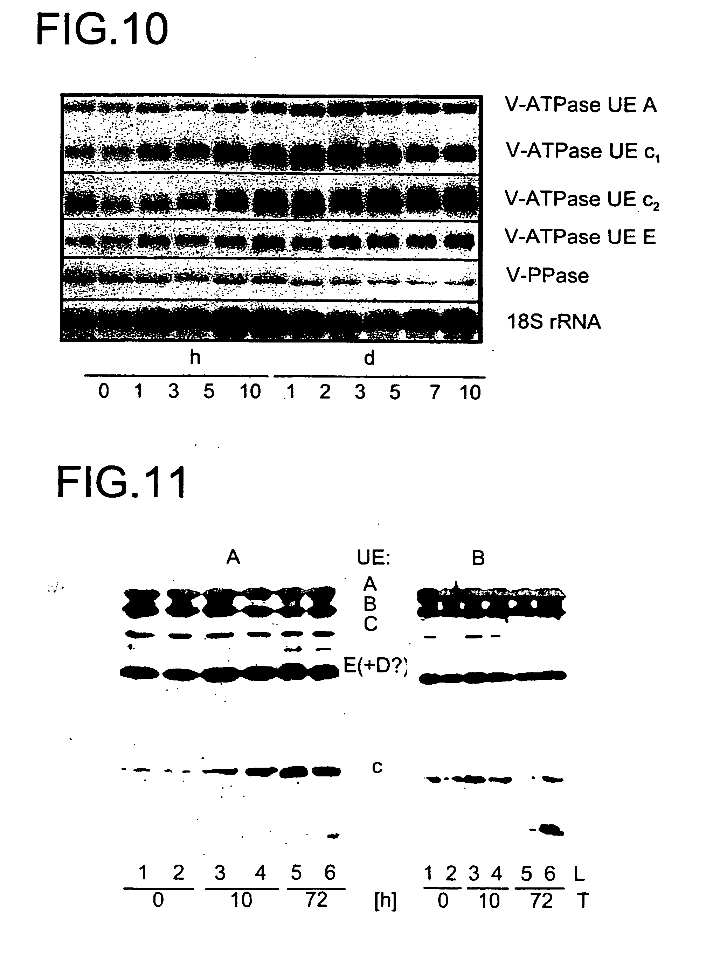

Zum Nachweis der steady-state-Transkriptspiegel der V-PPᵢase sowie verschiedener V-ATPase-Untereinheiten aus dem Kopf-, Stiel- und membranintegralen Bereich wurden zu mehreren Zeitpunkten nach Verwundung Proben genommen. Nach Isolierung der RNA wurde ein Northern Blot mit einer Auftragsmenge von 15 µg RNA pro Zeitpunkt angefertigt. Derselbe Blot wurde mehrfach nacheinander mit unterschiedlichen homologen Biotin-markierten Sonden entwickelt. Um die Transkripte der beiden Isoformen des Proteolipids c nachzuweisen, wurde die genspezifische c1-Sonde nach der Detektion von der Membran heruntergewaschen ("gestrippt"), bevor die Hybridisierung mit der genspezifischen c2-Sonde erfolgte. Wie in Figur 10 zu erkennen, wurde die c1-Sonde nicht vollständig heruntergewaschen; über den Signalen der Isoform c2 sind noch schwach die Banden des etwas größeren Transkripts der Isoform c1 zu erkennen, was andererseits beweist, daß sich die mRNAs der Isoformen tatsächlich spezifisch nachweisen ließen. Da die Transkripte der A-Untereinheit sowie der V-PPᵢase ebenfalls ähnlich weit im Gel laufen, wurde die Membran zwischen der Detektion der mRNA für die A-Untereinheit und für die V-PPᵢase erneut "gestrippt".

Die Speicherwurzel der Zuckerrübe ist ein Gewebe, das zur Aufrechterhaltung seiner Saccharosespeicherkapazität einer starken Energetisierung des Tonoplasten bedarf. Figur 10 zeigt, daß in der Tat beide vakuolären Protonenpumpen - wie nach der Neutralrotfärbung zu erwarten - eine signifikante Basisexpression aufweisen.

In Figur 10 ist die Northern Blot-Analyse zum Nachweis der Genexpression von V-ATPase und V-PPᵢase in Speicherparenchymzellen der Zuckerrübe nach mechanischer Verwundung gezeigt. Die RNA-Auftragsmenge betrug jeweils 15 µg.

### Wundinduzierte Veränderungen der V-ATPase auf Proteinebene

Ob sich die nach Verwundung im Northern Blot beobachteten erhöhten Transkriptionspegel zahlreicher V-ATPase-Gene auch in erhöhten Proteimnengen niederschlagen, wurde durch Western Blot-Analyse überprüft. Membranproteine sowohl der angereicherten Tonoplastenfraktion als auch der gesamt-mikrosomalen Fraktion, die 0, 10 und 72 h nach Verwundung isoliert worden waren, wurden dazu in einem 13%igen PAA-Gel elektrophoretisch aufgetrennt und auf eine PVDF-Membran geblottet. Die Membran wurde anschließend mit dem Antiserum gegen das Holoenzym der V-ATPase aus *K. daigremontiana* entwickelt. Es zeigt sich, daß bei der gesamt-mikrosomalen Fraktion keine signifikante quantitative Veränderung der V-ATPase-Untereinheiten zu beobachten ist (Figur 11B). Auch bei der angereicherten Tonoplastenfraktion bleiben die Proteinmengen der Untereinheiten nach Verwundung offenbar konstant - mit einer Ausnahme: Die Untereinheit c, deren Isoformen auch auf mRNA-Ebene die stärkste Induktion gezeigt hatten, war nach Verwundung deutlich erhöht. Diese Induktion war bei den angereicherten Tonoplastenfraktionen zweier Rüben, die unabhängig voneinander isoliert worden waren, gleich stark (Figur 11A).

Figur 11 zeigt die Western Blot-Analyse mit einem polyklonalen Antiserum gegen das Holoenzym der V-ATPase aus *K. daigremontiana* und offenbart wundinduzierte Veränderungen der V-ATPase am Tonoplasten im Speicherparenchym der Zuckerrübe. A. In der angereicherten Tonoplastenfraktion nimmt die Proteinmenge der Untereinheit c nach Verwundung zeitabhängig zu. B. In der gesamt-mikrosomalen Fraktion bleiben die Mengen der einzelnen Untereinheiten unverändert. Aufgetragen wurden jeweils 5 µg Protein von den isolierten Membranen zweier Zuckerrüben. Rübe 1: Bahn 1, 3 und 5; Rübe 2: Bahn 2, 4 und 6.

### Verminderte H⁺-Pumpaktivitiit der V-ATPase nach Verwundung

Wie in den vorangegangenen Abschnitten beschrieben, wurde nach Verwundung für mehrere V-ATPase-Gene eine Induktion auf mRNA-Ebene und durch Western Blot-Analyse ein erhöhter Proteinspiegel der Untereinheit c am Tonoplasten nachgewiesen. Es stellte sich daher die Frage, ob diese Veränderungen auch zu einer erhöhten Protonenpumpleistung der V-ATPase führen. Um dies zu untersuchen, wurde die H⁺-Pumpaktivitat isolierter Membranvesikel der mikrosomalen und der angereicherten Tonoplastenfraktion in Gegenwart der Hemmstoffe Vanadat für P-ATPasen und Azid für F-ATPasen mittels des Fluoreszenzfarbstoffes Acridinorange gemessen. Das Pumpmedium enthielt 250 mM Sorbitol anstelle von Saccharose, um durch den H⁺/Saccharose-Antiporter hervorgerufene Effekte auszuschließen.

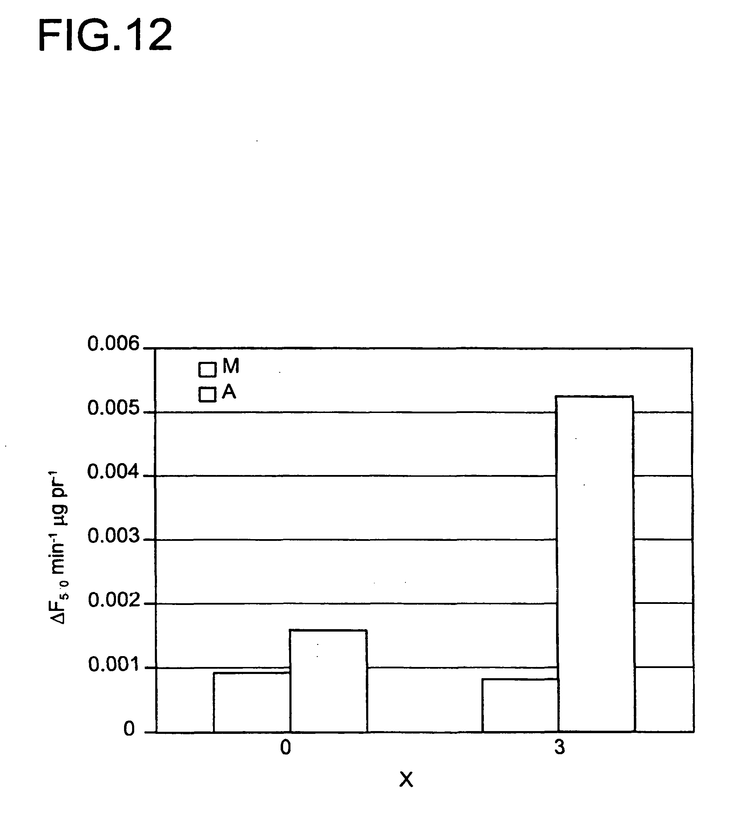

Wie Figur 12 zeigt, ist die H⁺-Pumpaktivität der V-ATPase in der mikrosomalen Fraktion vor und nach Verwundung gleich hoch. Dagegen kommt bei der angereicherten Tonoplastenfraktion 3 Tage nach Verwundung zu einer 3,3-fachen Erhöhung der Protonenpumpaktivät. Die nach Verwundung erhähten Proteinspiegel des Proteolipids c am Tonoplasten (Figur 12) sind demnach begleitet von einer signifikant erhöhten H⁺-Pumpaktivität der darin lokalisierten V-ATPase.

Figur 12 zeigt wundinduzierte Veränderungen der H⁺-Pumpaktivität der V-ATPase in der mikrosomalen und der angereicherten Tonoplastenfraktion in Gegenwart von 100 µM Vanadat und 1 mM Azid. Während die Anfangsrate der Fluoreszenzabnahme (ΔF540) Von Acridinorange nach Zugabe von 1 mM MgATP zu Vesikeln der mikrosomalen Fraktion (40 µg Protein) vor und nach Verwundung gleich hoch ist, kommt es bei der angereicherten Tonoplastenfraktion (10 µg Protein) zu einer 3,3-fachen Erhöhung. Gezeigt sind die Mittelwerte von jeweils 3 Einzelmessungen.

| Erklärung der in den Figuren benützen Abkürzungen: | | |
|---|---|---|
| Fig. 2 | **BVA-70 P** | BVA-70 Promotor |
| | **L** | Luciferase |
| | **X** | Die BVA-70 Promotor-LUC-CaMV-ter Kassette im pBluescript II SK + (3424bp) |
| Fig. 3 | **BVA-16/1 P** | BVA-16/1 Promotor |
| | **L** | Luciferase |
| | **X** | Die BVA16/1 Promotor-LUC-CaMV-ter Kassette im pBluescript II SK+ (3446bp) |
| Fig. 4 | **BVA/16-2 orig** | BVA/16-2 original |
| | **D** | Deletion |
| Fig. 5 | **BVA/16-1 orig** | BVA/16-1 original |
| | **D** | Deletion |
| Fig. 6 | **X** | Aktivitätsvergleich verschiedener Promotoren unter |
| | **L** | Kontrollbedingungen Luciferase |
| | **RLA** | Relative Luciferaseaktivität |
| Fig. 7 | **W** | Wurzel |
| | **J** | Junges Blatt |
| | **V** | Voll expandiertes Blatt |
| Fig. 9c | **co** | Kontrolle |
| | **su** | Untereinheit |
| Fig. 8A | **X** | Promotordeletionen von BVA/16-1 |
| | **Y** | relative Luciferaseaktivität |
| Fig. 8B | **X** | Promotordeletionen von BVA/16-2 |
| | **Y** | relative Luciferaseaktivität |
| Fig. 9A,B | **co** | Kontrolle |
| | **L** | Luciferase |
| Fig. 10 | **h** | Zeit nach Verwundung in Stunden [h] |
| | **d** | Zeit nach Verwundung in Tagen [d] |
| Fig. 11 | **L** | Bahn |
| | **T** | Zeit nach Verwundung in Stunden [h] |
| Fig. 12 | **M** | mirkrosomale Fraktion |
| | **A** | angereicherte Tonoplastenfraktion |
| | **pr** | Protein |
| | **X** | Zeit nach Verwundung in [h] |

## Patentansprüche

1. DNA-Konstrukt, umfassend die Nukleotidsequenz des pflanzlichen Promotors der *B. vulgaris* V-ATPase Untereinheit c Isoform 2 [Seq ID Nr. 1] operativ verbunden mit einem heterologen Gen.

2. DNA-Konstrukt nach Anspruch 1, **dadurch gekennzeichnet, dass** die Nukleotidsequenz des pflanzlichen Promotors der *B. vulgaris* V-ATPase Untereinheit c Isoform 2 [Seq ID Nr. 1] eine Deletion besitzt oder dass die Sequenz des Promotors ein Hybrid der *B. vulgaris* V-ATPase Untereinheit c Isoform 2 [Seq ID Nr. 1] ist, wobei die Promoter-Sequenz weiterhin funktionell als Promoter aktiv ist.

3. DNA-Konstrukt nach einem der Ansprüche 1 oder 2, **dadurch gekennzeichnet, dass** es einen auf andere Weise als den ersten Promotor regulierbaren zweiten Promotor umfaßt.

4. DNA-Konstrukt nach einem der Ansprüche 1 bis 3, **dadurch gekennzeichnet, dass** der pflanzliche Promotor zusätzlich zu seinen endogenen Pyrimidinstretch-Sequenzen mindestens einen weiteren Pyrimidinstretch besitzt.

5. DNA-Konstrukt nach einem der Ansprüche 1 bis 4, **dadurch gekennzeichnet, dass** das heterologe Gen ein Selektionsmarker oder Resistenz vermittelndes Gen oder ein Gen von sonstigem medizinischem oder agronomischem Interesse ist.

6. Nukleinsäure, umfassend die Sequenz des Promotors der *B. vulgaris* V-ATPase Untereinheit c Isoform 2 [SEQ ID Nr. 1].

7. Nukleinsäure nach Anspruch 6, **dadurch gekennzeichnet, dass** sie eine Deletion besitzt oder dass ihre Nukleotidsequenz ein Hybrid der *B. vulgaris* V-ATPase Untereinheit c Isoform 2 [Seq ID Nr. 1] ist, wobei die Nukleinsäure weiterhin funktionell als Promoter aktiv ist.

8. Rekombinanter Vektor, **dadurch gekennzeichnet, daß** er ein DNA-Konstrukt nach einem der Ansprüche 1 bis 5 umfaßt.

9. Rekombinanter Vektor nach Anspruch 8, **dadurch gekennzeichnet, daß** er ein Expressionsvektor ist.

10. Mikroorganismus, **dadurch gekennzeichnet, daß** er mit einem rekombinanten Vektor nach den Ansprüchen 8 oder 9 transformiert ist.

11. Transgene pflanzliche Zelle oder Protoplast, **dadurch gekennzeichnet, daß** ihr Genom ein DNA-Konstrukt nach einem der Ansprüche 1 bis 5 umfaßt.

12. Transgene Pflanze, **dadurch gekennzeichnet, daß** ihr Genom ein DNA-Konstrukt nach einem der Ansprüche 1 bis 5 umfaßt.

13. Verfahren zur gesteuerten Expression eines heterologen Gens in einer Pflanzenzelle oder einem Protoplasten, **dadurch gekennzeichnet, daß**
die Zelle oder der Protoplast mit einem DNA-Konstrukt nach einem der Ansprüche 1 bis 5 transformiert wird und
die so transformierte Zelle oder der Protoplast anschließend einem solchen biotischen oder abiotischen Streß ausgesetzt wird,
daß die Expression des mittels des DNA-Konstruktes transformierten heterologen Gens gesteuert wird.

14. Verfahren zur gesteuerten Expression eines heterologen Gens in einer Pflanze, **dadurch gekennzeichnet, daß**
mit einem DNA-Konstrukt nach einem der Ansprüche 1 bis 5 transformierte Zellen oder Protoplasten zu einer transgenen Pflanze regeneriert werden und
die so transformierte Pflanze anschließend einem solchen biotischen oder abiotischen Streß ausgesetzt wird,
daß die Expression des mittels des DNA-Konstruktes transformierten heterologen Gens gesteuert wird.

15. Verfahren zur Herstellung eines rekombinanten Proteins, **dadurch gekennzeichnet, daß**
eine Pflanzenzelle oder ein Protoplast mit einem DNA-Konstrukt nach einem der Ansprüche 1 bis 5 transformiert wird und
die so transformierte Zelle oder der Protoplast anschließend einem solchen biotischen oder abiotischen Streß ausgesetzt wird,
daß die Expression des mittels des DNA-Konstruktes transformierten rekombinanten Proteins erfolgt.

16. Verfahren zur Herstellung eines rekombinanten Proteins in einer Pflanze, **dadurch gekennzeichnet, daß**
mit einem DNA-Konstrukt nach einem der Ansprüche 1 bis 5 transformierte Zellen oder Protoplasten zu einer transgenen Pflanze regeneriert werden und
die so erhaltene transgene Pflanze anschließend einem solchen biotischen oder abiotischen Streß ausgesetzt wird,
daß die Expression des mittels des DNA-Konstruktes transformierten rekombinanten Proteins erfolgt.

17. Verwendung eines DNA-Konstrukts nach einem der Ansprüche 1 bis 5 zur Herstellung eines rekombinanten Proteins in einer Pflanzenzelle oder einem Protoplasten.

18. Verwendung eines DNA-Konstrukts nach einem der Ansprüche 1 bis 5 zur Herstellung eines rekombinanten Proteins in einer Pflanze.

19. Verwendung eines DNA-Konstrukts nach einem der Ansprüche 1 bis 5 zur Expression eines Gens in einer Pflanze unter biotischem oder abiotischem Streß.

20. Verwendung des pflanzlichen V-ATPase Promotors Untereinheit c Isoform 2 [Seq ID No. 1] zur Expression eines Gens in einer Pflanze unter biotischem oder abiotischem Streß.

21. Verwendung nach Anspruch 20, **dadurch gekennzeichnet, daß** die Nukleotidsequenz des pflanzlichen V-ATPase Promotors Untereinheit c Isoform 2 [Seq ID Nr. 1] eine Deletion besitzt oder daß die Sequenz des Promotors ein Hybrid der *B. vulgaris* V-ATPase Untereinheit c Isoform 2 [SEQ ID No. 1] ist.

22. Verwendung nach einem der Ansprüche 20 oder 21, **dadurch gekennzeichnet, daß** der pflanzliche Promotor zusätzlich zu seinen endogenen Pyrimidinstretch-Sequenzen mindestens einen weiteren Pyrimidinstretch besitzt.

23. Pflanzenzelle oder Protoplast, **dadurch gekennzeichnet, daß** sie mit einem DNA-Konstrukt nach einem der Ansprüche 1 bis 5 transformiert worden ist und daß sie gegen biotischen oder abiotischen Streß resistent ist.

24. Pflanzenzelle oder Protoplast nach Anspruch 23, **dadurch gekennzeichnet, daß** sie gegen Salzstreß resistent ist.

25. Pflanze, **dadurch gekennzeichnet, daß** sie mit einem DNA-Konstrukt nach einem der Ansprüche 1 bis 5 transformiert worden ist und daß sie gegen biotischen oder abiotischen Streß resistent ist.

26. Pflanze nach Anspruch 25, **dadurch gekennzeichnet, daß** sie gegen Salzstreß resistent ist.

## Claims

1. A DNA construct comprising the nucleotide sequence of the plant promoter of the *B. vulgaris* V-ATPase subunit c isoform 2 [SEQ ID No. 1], in operative linkage with a heterologous gene.

2. The DNA construct according to claim 1, wherein the nucleotide sequence of the plant promoter of the *B. vulgaris* V-ATPase subunit c isoform 2 [SEQ ID No. 1] has a deletion or wherein the sequence of the promoter is a hybrid of the *B. vulgaris* V-ATPase subunit c isoform 2 [SEQ ID No. 1], where the promoter sequence continues to be functionally active as promoter.

3. The DNA construct according to either of claims 1 and 2, which comprises a second promoter which can be regulated in a different manner than the first promoter.

4. The DNA construct according to any of claims 1 to 3, wherein the plant promoter has at least one further pyrimidine stretch in addition to its endogenous pyrimidine stretch sequences.

5. The DNA construct according to any of claims 1 to 4, wherein the heterologous gene is a selection marker or a resistance-mediating gene or a gene of any other medical or agronomic interest.

6. A nucleic acid comprising the sequence of the promoter of the *B. vulgaris* V-ATPase subunit c isoform 2 [SEQ ID No. 1].

7. The nucleic acid according to claim 6, which has a deletion or whose nucleotide sequence is a hybrid of the *B. vulgaris* V-ATPase subunit c isoform 2 [SEQ ID No. 1], where the nucleic acid continues to be functionally active as promoter.

8. A recombinant vector which encompasses a DNA construct according to any of claims 1 to 5.

9. A recombinant vector according to claim 8, which is an expression vector.

10. A microorganism which is transformed with a recombinant vector according to either of claims 8 and 9.

11. A transgenic plant cell or protoplast whose genome encompasses a DNA construct according to any of claims 1 to 5.

12. A transgenic plant whose genome encompasses a DNA construct according to any of claims 1 to 5.

13. A method for the controlled expression of a heterologous gene in a plant cell or a protoplast, which comprises
transforming the cell or the protoplast with a DNA construct according to any of claims 1 to 5 and
subsequently exposing the transformed cell or the protoplast to such a biotic or abiotic stress
that the expression of the heterologous gene which has been transformed by means of the DNA construct is controlled.

14. A method for the controlled expression of a heterologous gene in a plant, which comprises
regenerating cells or protoplasts transformed with a DNA construct according to any of claims 1 to 5 to give rise to a transgenic plant and subsequently exposing the plant transformed in this way to such a biotic or abiotic stress
that the expression of the heterologous gene which has been transformed by means of the DNA construct is controlled.

15. A method for producing a recombinant protein, which comprises
transforming a plant cell or a protoplast with a DNA construct according to any of claims 1 to 5 and
subsequently exposing the transformed cell or the protoplast to such a biotic or abiotic stress
that the recombinant protein transformed by means of the DNA construct is expressed.

16. A method of producing a recombinant protein in a plant, which comprises
regenerating cells or protoplasts transformed with a DNA construct according to any of claims 1 to 5 to give rise to a transgenic plant and
subsequently exposing the resulting transgenic plant to such a biotic or abiotic stress
that the recombinant protein transformed by means of the DNA construct is expressed.

17. The use of a DNA construct according to any of claims 1 to 5 for producing a recombinant protein in a plant cell or a protoplast.

18. The use of a DNA construct according to any of claims 1 to 5 for producing a recombinant protein in a plant.

19. The use of a DNA construct according to any of claims 1 to 5 for expressing a gene in a plant under biotic or abiotic stress.

20. The use of the plant V-ATPase promoter subunit c isoform 2 [SEQ ID No. 1] for the expression of a gene in a plant under biotic or abiotic stress.

21. The use according to claim 20, wherein the nucleotide sequence of the plant V-ATPase promoter subunit c isoform 2 [SEQ ID No. 1] has a deletion or wherein the sequence of the promoter is a hybrid of the *B. vulgaris* V-ATPase subunit c isoform 2 [SEQ ID No. 1].

22. The use according to either of claims 20 and 21, wherein the plant promoter has at least one further pyrimidine stretch in addition to its endogenous pyrimidine stretch sequences.

23. A plant cell or protoplast, which has been transformed with a DNA construct according to any of claims 1 to 5 and which is resistant to biotic or abiotic stress.

24. The plant cell or protoplast according to claim 23, which is resistant to salt stress.

25. A plant which has been transformed with a DNA construct according to any of claims 1 to 5 and which is resistant to biotic or abiotic stress.

26. The plant according to claim 25, which is resistant to salt stress.

## Revendications

1. Construction d'ADN, comportant la séquence nucléotidique du promoteur végétal de l'isoforme 2 de la sous-unité c de la V-ATPase de *B. vulgaris* [SEQ ID NO 1] reliée de manière opérationnelle à un gène hétérologue.

2. Construction d'ADN suivant la revendication 1, **caractérisée en ce que** la séquence nucléotidique du promoteur végétal de l'isoforme 2 de la sous-unité c de la V-ATPase de *B. vulgaris* [SEQ ID NO 1] présente une délétion ou **en ce que** la séquence du promoteur est un hybride de l'isoforme 2 de la sous-unité c de la V-ATPase de *B. vulgaris* [SEQ ID NO 1], la séquence de promoteur étant de plus fonctionnellement active comme promoteur.

3. Construction d'ADN suivant l'une des revendications 1 ou 2, **caractérisée en ce qu'**elle comporte un deuxième promoteur qui peut être régulé d'une autre manière que le premier promoteur.

4. Construction d'ADN suivant l'une des revendications 1 à 3, **caractérisée en ce que** le promoteur végétal présente, en supplément de ses séquences d'allongement à pyrimidine endogènes, au moins un autre allongement à pyrimidine.

5. Construction d'ADN suivant l'une des revendications 1 à 4, **caractérisée en ce que** le gène hétérologue est un marqueur de sélection ou un gène procurant une résistance ou un gène présentant un autre intérêt médical ou agronomique.

6. Acide nucléique comportant la séquence du promoteur de l'isoforme 2 de la sous-unité c de la V-ATPase de *B. vulgaris* [SEQ ID NO 1].

7. Acide nucléique suivant la revendication 6, **caractérisé en ce qu'**il présente une délétion ou **en ce que** sa séquence nucléotidique est un hybride de l'isoforme 2 de la sous-unité c de la V-ATPase de *B. vulgaris* [SEQ ID NO 1], l'acide nucléique étant de plus fonctionnellement actif comme promoteur.

8. Vecteur recombinant, **caractérisé en ce qu'**il comporte une structure d'ADN selon l'une des revendications 1 à 5.

9. Vecteur recombinant suivant la revendication 8, **caractérisé en ce qu'**il est un vecteur d'expression.

10. Micro-organisme, **caractérisé en ce qu'**il est transformé par un vecteur recombinant suivant les revendications 8 ou 9.

11. Cellule végétale ou protoplaste transgénique, **caractérisé en ce que** son génome comporte une construction d'ADN suivant l'une des revendications 1 à 5.

12. Plantes transgéniques, **caractérisées en ce que** leur génome comporte une construction d'ADN suivant l'une des revendications 1 à 5.

13. Procédé d'expression induite d'un gène hétérologue dans une cellule végétale ou un protoplaste, **caractérisé en ce que**
la cellule ou le protoplaste est transformé par une construction d'ADN suivant l'une des revendications 1 à 5, et
la cellule ou le protoplaste ainsi transformé est ensuite exposé à un stress biotique ou abiotique tel que l'expression du gène hétérologue transformé au moyen de la construction d'ADN est induite.

14. Procédé d'expression induite d'un gène hétérologue dans une plante, **caractérisé en ce que**
des cellules ou protoplastes transformés par une construction d'ADN suivant l'une des revendications 1 à 5 sont régénérés en une plante transgénique et
la plante ainsi transformée est ensuite soumise à un stress biotique ou abiotique
tel que l'expression du gène hétérologue transformé au moyen de la construction d'ADN soit induite.

15. Procédé de préparation d'une protéine recombinante, **caractérisé**
**en ce qu'**on transforme une cellule végétale ou un protoplaste avec une construction d'ADN suivant l'une des revendications 1 à 5, et
on soumet ensuite la cellule ou le protoplaste ainsi transformé à un stress biotique ou abiotique tel que l'expression de la protéine recombinante transformée au moyen de la construction d'ADN a lieu.

16. Procédé de préparation d'une protéine recombinante dans une plante, **caractérisé**
**en ce qu'**on régénère des cellules ou protoplastes transformés avec une construction d'ADN suivant l'une des revendications 1 à 5 en une plante transgénique, et
on soumet ensuite la plante transgénique ainsi obtenue à un stress biotique ou abiotique
tel que l'expression de la protéine recombinante transformée au moyen de la construction d'ADN a lieu.

17. Utilisation d'une construction d'ADN suivant l'une des revendications 1 à 5, pour la préparation d'une protéine recombinante dans une cellule végétale ou un protoplaste.

18. Utilisation d'une construction d'ADN suivant l'une des revendications 1 à 5, pour la préparation d'une protéine recombinante dans une plante.

19. Utilisation d'une construction d'ADN suivant l'une des revendications 1 à 5, pour l'expression d'un gène dans une plante sous stress biotique ou abiotique.

20. Utilisation de l'isoforme 2 de la sous-unité c du promoteur de la V-ATPase végétal [SEQ ID NO 1], pour l'expression d'un gène dans une plante sous stress biotique ou abiotique.

21. Utilisation suivant la revendication 20, **caractérisée en ce que** la séquence nucléotidique de l'isoforme 2 de la sous-unité c du promoteur de la V-ATPase végétal [SEQ ID NO 1] présente une délétion ou **en ce que** la séquence du promoteur est un hybride de l'isoforme 2 de la sous-unité c de la V-ATPase de *B. vulgaris* [SEQ ID NO 1].

22. Utilisation suivant l'une des revendications 20 et 21, **caractérisée en ce que** le promoteur végétal présente, en supplément de ses séquences d'allongement à pyrimidine endogènes, au moins un autre allongement à pyrimidine.

23. Cellule végétale ou protoplaste, **caractérisé en ce qu'**il a été transformé par une construction d'ADN suivant l'une des revendications 1 à 5 et **en ce qu'**il est résistant au stress biotique ou abiotique.

24. Cellule végétale ou protoplaste suivant la revendication 23, **caractérisé en ce qu'**il est résistant à un stress salin.

25. Plante, **caractérisée en ce qu'**elle a été transformée par une construction d'ADN suivant l'une des revendications 1 à 5 et **en ce qu'**elle est résistante au stress biotique ou abiotique.

26. Plante suivant la revendication 25, **caractérisée en ce qu'**elle est résistante au stress salin.


### SEQUENZPROTOKOLL

<110> BASF AG
<120> Pflanzliche Genexpression unter der Kontrolle von konstitutiven pflanzlichen V-ATPase Promotoren
<130> BASF - NAE 1074/99
<140>
   <141>
<160> 3
<170> Patentln Ver. 2.1
<210> 1
   <211> 2090
   <212> DNA
   <213> Beta vulgaris
<220>
   <223> Promotor subunit c isoform 2
<220>
   <221> promoter
   <222> (1)..(1923)
<400> 1
   gatatcacac attcgtccat cgacgatttg cgaactttca aataggtacg taattctttt 60
   aatcttcaaa gttatttcac attagtcgac tattatgtag ttgaaaaaat ggagataata 120
   ggaattagtt gaaaagggtg tttatataat tagacttaaa tttgattcat tttcatatat 180
   ctgaaaacaa ggtatgtatg aaatttgatt catttatgac actgatgaaa aagttaacga 240
   tttagttctt ttttttaaaa ttccaatata aatttttgcc caaaactttt gcaaaatatc 300
   catgttcgga aataaatttt gaaaaacaaa acaatatcaa acctttttgc gaacaacttt 360
   tacaaaaatc cattttcaga aaaaaaaatt tacattaact tgcgaaatca aattgtgtat 420
   gaaaaattta aaatttcctt tcacctataa ttgaaactca aagtgttaaa atttagaaaa 480
   ggagaaaaat aaaaaatgac catttcatgc gaaatcaaat tgtgtatgaa aaacttaaaa 540
   ttttatttta aatataattg aaattcaaag tgttaaaatt tagaaaagga gaaaaattaa 600
   aatgaccatt tcattcaaaa tcagattgtg tatgaaaaat ttaaattttt atttcaaata 660
   taattgaaac tcaaagtgtg aacatttaga aaaggagaaa aattaaaatg atgaaaattt 720
   gtaaaacatc aatttgtgaa atcagaattt agaagttaga caaggaaaaa aaactgaatt 780
   gtcttatact tttcggttac aattttggga tcataaagaa attactgaaa tccatatcaa 840
   aaactattat aaattacaaa aatgaataaa accaaaaaaa gaagaacatg acgatatttc 900
   gtaaagaaca tcatactgat tataaaagaa catgcgcata ttagaattga gaaacaaaaa 960
   actattcaaa atcacaaaaa tggataacaa catacaaaga acatgaaaga atcttattca 1020
   caaaatggag gtgaacttaa atactaactt gcattttcag tttatttact acttagtatt 1080
   aggcctaaaa atatcatcgc acgcatcgcg tgcacaaaag actagtgtta agtatcacaa 1140
   gtcacaaact cacaactgat tttcatttag gctccatttg gtagggcgta aaacgttttc 1200
   ccggaacact atttttctcc atttttagtt ttacattgtt tggttgacaa aagagtgtaa 1260
   aaccgttttc ccttggggta aaattactct tccaatgatc gaaaaccatt ttcctttcaa 1320
   aatgaaggga aaactgtttt ccttatctct cttgttacac ttttctcact acctccttac 1380
   tttccctttt attttacttt catttcatca ttttctttgc atgaaaccaa acaacggaaa 1440
   actaattttg gaattgtgtt ttccattgta aattgttttc catgaaaatc attttacact 1500
   gaaaatgttt tacaccctac caaacagagc cttcgtgtgc catgaatgca tgaccgattt 1560
   caaattcgaa actggtgttt accgtttcct aattggtttc gagttcacaa ccaagtattc 1620
   aactatgttg ctcaccctaa agatgaatat ggtaaaacct tgaggtgggc tttggctaaa 1680
   aaaagtccca ccaagcccca attctaggct cccaaaacca cgaaatttcc tggtactatt 1740
   ccaaaacaaa aacaaacacc tcctgatcaa accagaaaaa ataaaacata tttttgtttt 1800
   ctcccaattt ttcattttaa tttatcacgg gaaagtacac aataattcaa tcaagggtaa 1860
   aaaaaataaa ataaaaagaa aagataagta taaacaaaag aaatttgtct tctctacatc 1920
   ctcatatttc atccacgctc tcttcttcct ctcctctcat ctcctttttc aattcccaga 1980
   tcggatcaag caattcatcg aacaccttcc gatcatcacc atcaaaaaaa atgtcaacag 2040
   tctttaacgg cgatgaaacg gcgccgttct tcggctttct gggtgctgct 2090
<210> 2
   <211> 1153
   <212> DNA
   <213> Beta vulgaris
<220>
   <223> Promotor subunit c isoform 1
<300>
   <302> cDNA and genomic cloning of sugar beet V-type H+-ATPase subunit A and c isoforms: evidence for coordinate expression during plant development and coordinate induction in response to high salinity
   <303> Plant Mol. Biol.
   <304> 1999
   <305> 39
   <306> 463-475
<400> 2
   gggtgggggt gaagtggggg ctgcaggggt gggtgggggg ggctggtggc aggtattggt 60
   agtggtgcta ggtggtgatt atggaggaag aagggaaata gaagtggtta acaccgaaaa 120
   gtgagattaa gttttatctt gcaaaaataa tttatttttt ggtttgtttt cacaaagaat 180
   gctttattaa ggtttttttt tgcaaaaaac tgaactttta aaaggttttt tttgataaat 240
   tttcctaata ttaataaatg aaaatttttc taacatggga gatagcctgg acctataact 300
   agttgttata acttataaga agagaatttt gggcaactat gcggaaaata ggggaaattt 360
   tggaaaaaat aagtaaaaga ttttaggtgt aaacttgctt gttataagtt ataaacttga 420
   atttgttcta ttgatagtgt tggttaataa tgtgacaact aaatcgaaca taaaggttta 480
   ggaccctttc gacatcattg agagttagag gtgggcatga gccgggccag ttcgcgtgct 540
   ggaacggcca gttcgcgggc tggtacggcc aggcacgagc ccgatcaagg cacgacccgg 600
   cttgttgtcc gctacgtgga ccgtgggccc atcacggtca aaaaaaaata ttttaggctc 660
   gcaccagcac gagcccgcgt aggccagccc gcgggctgaa tgttcctttc ctttgttttt 720
   tagcctaaaa ctgtcataaa tacaaaaaga taacataaaa cacaggctag gccctcaata 780
   ggcacgagcc cgtggaaaaa atccgaaaat tccaagccta gctagttact cacaagctca 840
   gacccgattc gtcatttttc caaacccgtg ggccgagccg gtgaacaggc tcggcccggc 900
   tcatgtccag gtgacaggtc tactgagagt tatgagttgt ttccccatgg gctacttgac 960
   tacaaacctt ccatagttcc atgacacttc catctgagcc catgagataa aattaaggaa 1020
   tacaaacaca aaactcaaca accaacccga aatatcgatc ggacttgtta gcacgtgtta 1080
   tgttgggtcc catccaagaa actttctctc tcctcctcct tcataaaaaa accttctcac 1140
   tgatcccatc cag 1153
<210> 3
   <211> 1073
   <212> DNA
   <213> Beta vulgaris
<220>
   <223> Promotor subunit A
<300>
   <302> cDNA and genomic cloning of sugar beet V-type H+-ATPase subunit A and c isoforms: evidence for coordinate expression during plant development and coordinate induction in response to high salinity
   <303> Plant Mol. Biol.
   <304> 1999
   <305> 39
   <306> 463-475
<400> 3
   gaattctctg ctctggattc gacatcaaaa tttccaccgg gttactattc aaacagactg 60
   ctcaaatctc atttcttctc ttcgttctat ttcgacggtg cctcgctcta tctactggtc 120
   tgtatgccag attatgagtc taacaggaaa tcttcactgg tgttttctgc ttaaagtaaa 180
   ttgtcaaaaa gttcaaaagg cacaaaatgt ggctaagcta tgtgctcaaa tttgttgttt 240
   actacttgat ttgttttctt tctctttatg cctgtttttt ccccctttgt aaaaaaaaga 300
   tacaagtata gatgaaggga ttctttatat tggccccatg tgttcgttag ataattacat 360
   tgtaccctca attcctcatc ttccttgaag ttctacgtag taccattgtg gttgcataag 420
   caaatgataa tcatactttc atatcttagt taatgtacat cgtcacttgt gcttaacatg 480
   tcataaacta atttccttgg tttaatactg gttacattaa ctaaatcttt tattcttaaa 540
   tatttaagaa gtgtgcagta aattaagttt cttccaaatc ctcaataaga cattatactt 600
   agaaaacttc tataaagtta cttatcaact tacagataac gaccaaagaa tcatcaccaa 660
   aacagttatc gaaccacata gaagctgcat agcttttgaa aaaggtgaag gtacaatata 720
   aatctccaac aaatatagtg tatctactcc caaaagctat cctagtaata tccctatctc 780
   aaaaacatat cttttatcaa ctttttccca acacaaactc aattgttaaa aactacaagg 840
   aaacgttgtt taaccaatca ctatatttac attaaccata tttttaattt agttaaacct 900
   ctcaagtctc actacattct taaaaaaagt tggagataga gtcatcataa ttcatagaga 960
   aggaattgac aacatctaat aagaacgaat tacgaacgtg gcaaaatcac agacgaaaca 1020
   tagcaaaact taaccctgca aatctcaatc agatttaaat aatccttttg ctg 1073